# EUROPEAN PATENT APPLICATION

(11) **EP 4 406 952 A1**
(43) Date of publication of application: **31.07.2024**
(21) Application number: 23382068.7
(22) Date of filing: 27.01.2023
(51) Int. Cl.: C07D 413/06, A61K 31/4245, A61P 31/04

(54) **NEW COMPOUNDS FOR TREATING STAPHYLOCOCCUS INFECTIONS**

(71) Applicant: ABAC THERAPEUTICS, S.L., 08950 Esplugues de Llobregat (Barcelona) (ES)
(72) Inventor: TORRENS JOVER, Antoni, 08021 Terrassa (Barcelona) (ES); GARGALLO VIOLA, Domingo, 08860 Castelldefels (Barcelona) (ES); CAAMAÑO MOURE, Ana María, 15707 Santiago de Compostela (A Coruña) (ES); LLORENTE FERNÁNDEZ, Ana Virginia, 08902 Hospitalet de Llobregat (Barcelona) (ES)
(74) Representative: Herrero & Asociados, S.L.

(57) **Abstract**

The present invention relates to new antibacterial compounds of formula (I) useful against *Staphylococcus sp.* infections, especially against infections of *Staphylococcus aureus.* The invention is also directed to the process for the preparation of said antibacterial compounds, to compositions comprising them, and to their use as medicaments.

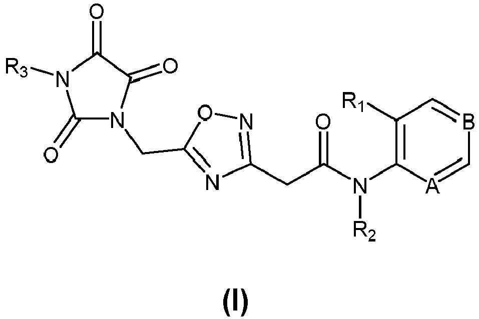

## Description

### FIELD OF THE INVENTION

The present invention relates to new antibacterial compounds useful against *Staphylococcus sp.* infections, especially against infections of *Staphylococcus aureus.* The invention is also directed to the process for the preparation of said antibacterial compounds, to compositions comprising them, and to their use as medicaments.

### BACKGROUND OF THE INVENTION

Infectious diseases are the second most important cause of human death worldwide and represent a significant global burden affecting society. Most of these diseases are due to exposure to or the invasion of host cells and organs by microorganisms. These pathogens disrupt the normal function of the human body by hindering immune responses and producing harmful toxins. Infectious diseases can easily spread from person-to-person *via* contact with body fluids, indirect contact or through animal vectors such as mosquitoes and ticks.

*Staphylococcus aureus* (S. aureus) is a very common human pathogenic microorganism that can trigger a variety of infectious diseases, such as skin and soft tissue infections, endocarditis, osteomyelitis, bacteremia, and lethal pneumonia. It is considered the 2nd pathogen in number of deaths in the world. *Staphylococcus aureus* is a Gram-positive type of bacteria and a non-moving small round shaped or non-motile cocci. It is found in grape-like clusters. For this reason, it is called *Staphylococcus.* It was discovered in Aberdeen, Scotland in 1880 in pus from surgical abscesses. *S*. *aureus* is considered the classic opportunist in this regard, taking advantage of broken skin or other entry sites to cause infection. In culture, bacterial colonies have a characteristic shiny, opaque, yellow to white appearance on blood agar. *S*. *aureus* belongs to the family Staphylococcic, and it affects all known mammalian species, including humans. Further due to its ability to affect a wide range of species, *S*. *aureus* can be readily transmitted from one species to another. This includes transmission between humans and animals.

*Staphylococcus* is one of the five most common causes of infections after injury or surgery. It affects around 500,000 patients in American hospitals annually. *S*. *aureus* may occur commonly in the environment, and it is transmitted through air droplets or aerosol. When an infected person coughs or sneezes, he or she releases numerous small droplets of saliva that remain suspended in air. These contain the bacteria and can infect others. Another common method of transmission is through direct contact with objects that are contaminated by the bacteria or by bites from infected persons or animals. Approximately 30% of healthy humans carry *S*. *aureus* in their nose, back of the throat and on their skin.

In normal healthy and immunocompetent person, *S*. *aureus* colonization of the skin, intestinal tract, or nasopharynx does not lead to any symptoms or disease but in animals and humans that are immunocompromised or immunodeficient, these bacteria may be life threatening. It may lead to pyogenic (abscessing) infections of the skin, eyes, and genital tract. When *S*. *aureus* is isolated from an abscess or boil or other skin lesion, it is usually due to its secondary invasion of a wound rather than the primary cause of disease. *S*. aureus may similarly be isolated from abscesses, breast abscesses or mastitis, dermatitis or skin infections and genital tract infections.

Of the variety of manifestations, *S*. *aureus* can cause minor skin infections, such as pimples, impetigo, furuncles (boils), cellulitis, folliculitis, carbuncles... It is also the cause of scalded skin syndrome and abscesses. Furthermore, *S*. *aureus* can cause lung infections or pneumonia, brain infections or meningitis, bone infections or osteomyelitis, heart infections or endocarditis, life-threatening generalized blood infections or toxic shock syndrome (TSS), bacteremia, and septicemia.

Beta-lactam antibiotics are the first-line treatment for Staphylococcal infections. Alternative, drugs are available for treatment but with limitations such as less tissue penetration and efficiency for vancomycin; cost for quinupristin, dalfopristin, tigecycline, daptomycin and linezolid and toxicity for rifampin.

Development of resistance to antibiotics has become a global issue and *S*. *aureus* owing to the selective pressure imposed by antimicrobials has the capability of developing resistance to drugs with more rapidity. According to the sensitivity to antibiotic drugs, S. *aureus* can be divided into methicillin-sensitive Staphylococcus aureus (MSSA) and methicillin-resistant Staphylococcus aureus (MRSA). In recent decades, due to the evolution of bacteria and the abuse of antibiotics, the drug resistance of S. aureus has gradually increased, the infection rate of MRSA has increased worldwide, and the clinical anti-infective treatment for MRSA has become more difficult. Accumulating evidence has demonstrated that the resistance mechanisms of S. aureus are very complex, especially for MRSA, which is resistant to many kinds of antibiotics. Therefore, understanding the drug resistance of MRSA in a timely manner and elucidating its drug resistance mechanism at the molecular level are of great significance for the treatment of *S*. *aureus* infection.

One of the alternative therapies studied aimed to minimize the development of resistance is the use of combinations of known antibiotics in the hope of a synergistic effect on resistant bacterial strains. However, this approach shows limitations in the choice of possible combinations and the long-term applicability of this strategy.

A second strategy aims to increase the sensitivity of bacterial strains to known antibiotics.

A third strategy consists in discovering new compounds capable of selectively controlling the bacteria Staphylococcus aureus. This approach has several advantages compared to the use of broad-spectrum antibiotics, because selective agents allow to kill or inhibit only those bacteria species that are causing the disease. As such, it leaves most of the beneficial bacteria unaffected, hence minimizing the collateral damage on the microbiota. Moreover, they have low propensity for bacterial resistance development.

For all these reasons, there is a real unmet need to find new selective compounds active against Staphylococcus aureus that allow to treat the disease effectively, avoiding the disadvantages of the use of broad-spectrum agents.

### SUMMARY OF THE INVENTION

The present invention discloses novel compounds with a potent antibiotic activity against *Staphylococcus sp.* and more particularly against *Staphylococcus aureus.* The compounds of the invention can be useful for the treatment of infections caused by these pathogens.

The invention is directed in a main aspect to a compound of Formula (I), 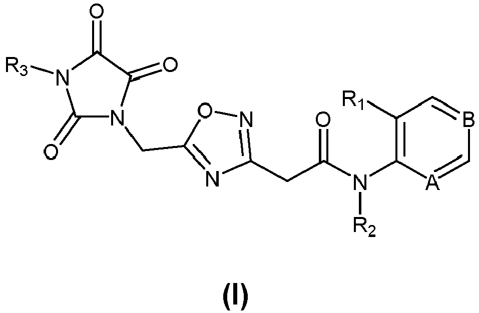 wherein **A**, **B**, **R₁**, **R₂** and **R₃** are as defined below in the detailed description.

A further aspect of the invention refers to the processes for preparation of compounds of formula (I).

It is also an aspect of the invention a pharmaceutical composition comprising a compound of formula (I).

Finally, it is an aspect of the invention a compound of formula (I) for use in therapy and more particularly for the treatment of bacterial infections caused *Staphylococcus sp,* particularly by *Staphylococcus aureus.*

### DETAILED DESCRIPTION OF THE INVENTION

The invention is directed to a family of compounds, in particular, showing a potent antibacterial activity against *Staphylococcus sp* thus, solving the above problem of identifying alternative compounds selectively controlling *Staphylococcus* species but avoiding the disadvantages of having a broad-spectrum.

The applicant has found that the problem of providing a new effective and alternative solution for preventing and treating *Staphylococcus* infections can surprisingly be solved by using compounds of the present inventions.

In a first aspect, the present invention is directed to a compound of formula (I): 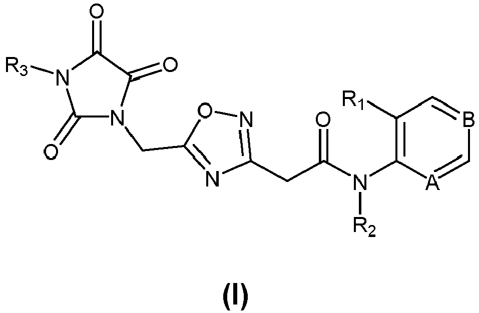 wherein:
**A** represents -CRₐ- or -N-;
**B** represents -CR_{b}- or -N-;
   **Rₐ** and **R_{b}** independently represent a hydrogen atom; a halogen atom; -CN; a branched or unbranched C₁₋₆ alkyl radical; a branched or unbranched C₁₋₆ haloalkyl radical, an -O**R_{ab}** radical, or a C₃₋₉ cycloalkyl radical;
   **R_{ab}** is a hydrogen atom or a branched or unbranched C₁₋₆ alkyl radical
**R₁** is a hydrogen atom; a halogen atom; a branched or unbranched C₁₋₆ alkyl radical or - O**R₁ₐ**;
   **R₁ₐ** is a hydrogen atom or a branched or unbranched C₁₋₆ alkyl radical;
**R₂** is a hydrogen atom; a branched or unbranched C₁₋₆alkyl radical optionally substituted; a halogen atom; a -CN; a C₁₋₆ haloalkyl radical; a -(CH₂)ₙ-OR₂ₐ; a -C(O)R_{2b}; an optionally substituted -(CH₂)ₘ-C₃₋₉ cycloalkyl; an optionally substituted -(CH₂)_{q}-C₃₋₉ heterocycloalkyl containing at least one heteroatom selected from N, O or S; an optionally substituted -(CH₂)ᵣ-heteroaryl containing at least one heteroatom selected from N, O or S; an optionally substituted -(CH₂)ₛ-aryl; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, -NO₂,=O, -OR_{2'}, -NR_{2'}R_{2"}, NR_{2'}C(O)R_{2"}, -NR_{2'}S(O)₂R_{2"}, - S(O)₂NR_{2'}R_{2"}, -NR_{2'}C(O)NR_{2"}R_{2‴}, -SR_{2'} , -S(O)R_{2'}, S(O)₂R_{2'}, -C(O)OR_{2'}, -C(O)NR_{2'}R_{2"},-OCH₂CH₂OH, -NR_{2'}S(O)₂NR_{2"}R_{2‴}, -C(CH₃)₂OR_{2'};
   **R_{2'}**, **R_{2'}**, and **R_{2‴}** are independently selected from a hydrogen atom or branched or unbranched C₁₋₆ alkyl radical;
**R₂ₐ** represent a hydrogen atom; a branched or unbranched C₁₋₆ alkyl radical optionally substituted with at least an R_{2a'}; a branched or unbranched C₁₋₆ haloalkyl radical, or a C₃₋₉ cycloalkyl radical;
   **R_{2a'}** a hydrogen atom or -OH;
**R_{2b}** independently represent a hydrogen atom; a halogen atom; -CN; a branched or unbranched C₁₋₆ alkyl radical; a branched or unbranched C₁₋₆ haloalkyl radical, an -OR_{2b}' radical, or a C₃₋₉ heterocycloalkyl radical optionally substituted by at least one -R_{2b"};
   **R_{2b'}** and **R_{2b"}** are independently selected from a hydrogen atom or branched or unbranched C₁₋₆ alkyl radical;
**R₃** is a hydrogen atom, an optionally substituted branched or unbranched C₁₋₆ alkyl; an optionally substituted -(CH₂)ᵢ-C₃₋₉ cycloalkyl; an optionally substituted -(CH₂)ⱼ-C₃₋₉ heterocycloalkyl containing at least one heteroatom selected from N, O or S; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, -NO₂,=O, -OR_{3'}, -NR₃,R_{3"}, NR_{3'}C(O)R_{3"}, -NR_{3'}S(O)₂R_{3"}, -S(O)₂NR_{3'}R_{3"}, -NR_{3'}C(O)NR_{3"}R_{3‴}, -SR_{3'} , -S(O)R_{3'}, S(O)₂R_{3'}, -C(O)OR_{3'},-C(O)NR_{3'}R_{3"}, -OCH₂CH₂OH, -NR₃,S(O)₂NR_{3"}R_{3‴}, -C(CH₃)₂OR_{3'};
   **R_{3'}**, **R_{3"}**, and **R_{3‴}** are independently selected from a hydrogen atom or branched or unbranched C₁₋₆ alkyl radical;
**n** is 0, 1, 2 or 3;
**m** is 0, 1, 2 or 3;
**q** is 0, 1, 2 or 3;
**r** is 0, 1, 2 or 3;
**s** is 0, 1, 2 or 3;
**i** is 0, 1, 2 or 3;
**j** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

Unless otherwise stated, the compounds of the invention are also meant to include isotopically labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The compounds of general formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

For the sake of clarity, the expression like "a compound according to formula (I), wherein R₁, R₂ and R₃ are as defined below in the detailed description" would (just like the expression "a compound of formula (I) as defined in the claims) refer to "a compound according to formula (I)", wherein the definitions of the respective substituents R₁ etc. (also from the cited claims) are applied.

For clarity purposes, all groups and definitions described in the present description and referring to compounds of formula (I), also apply to all intermediates of synthesis.

"Halogen" or "halo" as referred in the present invention represent fluorine, chlorine, bromine or iodine. When the term "halo" is combined with other substituents, such as for instance "C₁₋₆ haloalkyl" or "C₁₋₆ haloalkoxy" it means that the alkyl or alkoxy radical can respectively contain at least one halogen atom.

"C₁₋₆ alkyl", as referred to in the present invention, are saturated aliphatic radicals. They may be unbranched (linear) or branched and are optionally substituted. C₁₋₆₋alkyl as expressed in the present invention means an alkyl radical of 1, 2, 3, 4, 5 or 6 carbon atoms. Preferred alkyl radicals according to the present invention include but are not restricted to methyl, ethyl, propyl, n-propyl, isopropyl, butyl, n-butyl, tert-butyl, isobutyl, sec-butyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl. The most preferred alkyl radical are C₁₋₄ alkyl, such as methyl, ethyl, propyl, n-propyl, isopropyl, butyl, n-butyl, tert-butyl, isobutyl, sec-butyl, 1-methylpropyl, 2-methylpropyl or 1,1-dimethylethyl. Alkyl radicals, as defined in the present invention, are optionally mono- or polysubstituted by substitutents independently selected among others from a halogen, branched or unbranched C₁₋₆-alkoxy, branched or unbranched C₁₋₆-alkyl, C₁₋₆₋haloalcoxy, C₁₋₆-haloalkyl, trihaloalkyl or a hydroxyl group.

"C₁₋₆ alkoxy" as referered to in the present invention, is understood as meaning an alkyl radical as defined above attached via oxygen linkage to the rest of the molecule. Examples of alkoxy include, but are not limited to methoxy, ethoxy, propoxy, butoxy or tert-butoxy.

"C₃₋₆ Cycloalkyl" as referred to in the present invention, is understood as meaning saturated and unsaturated (but not aromatic), cyclic hydrocarbons having from 3 to 6 carbon atoms which can optionally be unsubstituted, mono- or polysubstituted. Examples for cycloalkyl radical preferably include but are not restricted to cyclopropyl, cyclobutyl, cyclopentyl, cyclohexyl. Cycloalkyl radicals, as defined in the present invention, are optionally mono- or polysubstituted by substitutents independently selected among others from a halogen atom, branched or unbranched C₁₋₆-alkyl, branched or unbranched C₁₋₆-alkoxy, C₁₋₆₋haloalcoxy, C₁₋₆-haloalkyl, trihaloalkyl or a hydroxyl group.

A heterocyclyl radical or group (also called heterocyclyl hereinafter) is understood as meaning 4 to 18 membered mono or fused polycyclic heterocyclic ring systems, with at least one saturated or unsaturated ring which contains one or more heteroatoms selected from the group consisting of nitrogen, oxygen and/or sulfur in the ring. A heterocyclic group can also be substituted once or several times.

Subgroups inside the heterocyclyls as understood herein include heteroaryls and non-aromatic heterocyclyls.
- the heteroaryl (being equivalent to heteroaromatic radicals or aromatic heterocyclyls) is an aromatic 5 to 18 membered mono or fused polycyclic heterocyclic ring system of one or more rings of which at least one aromatic ring contains one or more heteroatoms from the group consisting of nitrogen, oxygen and/or sulfur in the ring; preferably it is a 5 to 18 membered mono or fused polycyclic aromatic heterocyclic ring system of one or two rings of which at least one aromatic ring contains one or more heteroatoms selected from the group consisting of nitrogen, oxygen and/or sulfur in the ring; more preferably it is selected from furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, oxadiazole, thiophene and benzimidazole;
- the non-aromatic heterocyclyl is a 4 to 18 membered mono or fused polycyclic heterocyclic ring system of one or more rings of which at least one ring - with this (or these) ring(s) then not being aromatic - contains one or more heteroatoms from the group consisting of nitrogen, oxygen and/or sulfur in the ring; preferably it is a 4 to 18 membered mono or fused polycyclic heterocyclic ring system of one or two rings of which one or both rings - with this one or two rings then not being aromatic - contain/s one or more heteroatoms selected from the group consisting of nitrogen, oxygen and/or sulfur in the ring, more preferably it is selected from azetidine, oxetane, tetrahydrofuran, oxazepam, pyrrolidine, piperidine, piperazine, tetrahydropyran, morpholine, indoline, oxopyrrolidine, benzodioxane, especially is piperazine, benzodioxane, morpholine, tetrahydropyran, piperidine, oxopyrrolidine and pyrrolidine.

Preferably, in the context of this invention heterocyclyl is defined as a 4 to 18 membered mono or fused polycyclic ring system of one or more saturated or unsaturated rings of which at least one ring contains one or more heteroatoms selected from the group consisting of nitrogen, oxygen and/or sulfur in the ring. Preferably it is a 4 to 18 membered mono or fused polycyclic heterocyclic ring system of one or two saturated or unsaturated rings of which at least one ring contains one or more heteroatoms selected from the group consisting of nitrogen, oxygen, and sulfur in the ring. More preferably, it is a 4 to 12 membered mono or bicyclic heterocyclyl ring system containing one nitrogen atom and optionally a second heteroatom selected from nitrogen and oxygen. In another preferred embodiment of the invention, said heterocyclyl is a substituted mono or bicyclic heterocyclyl ring system.

Preferred examples of heterocyclyls include azetidine, azepane, oxetane, tetrahydrofuran, oxazepam, pyrrolidine, imidazole, oxadiazole, tetrazole, pyridine, pyrimidine, piperidine, piperazine, benzofuran, benzimidazole, indazole, benzodiazole, thiazole, benzothiazole, tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, tetrahydroisoquinoline, phthalazine, benzo-1,2,5-thiadiazole, indole, benzotriazole, benzoxazole oxopyrrolidine, pyrimidine, benzodioxolane, benzodioxane, carbazole and quinazoline, 3,9-diazaspiro[5.5]undecane, 2,8-diazaspiro[4.5]decane, 2,7-diazaspiro[3.5]nonane, 2,7-diazaspiro[4.4]nonane, octahydropyrrolo[3,4-c]pyrrole, especially is pyridine, piperazine, pyrazine, indazole, benzodioxane, thiazole, benzothiazole, morpholine, tetrahydropyran, pyrazole, imidazole, piperidine, thiophene, indole, benzimidazole, pyrrolo[2,3-b]pyridine, benzoxazole, oxopyrrolidine, pyrimidine, oxazepane, pyrrolidine, azetidine, azepane, oxetane, tetrahydrofuran, 3,9-diazaspiro[5.5]undecane, 2,8-diazaspiro[4.5]decane and 2,7-diazaspiro[3.5]nonane.

An N-containing heterocyclyl is a heterocyclic ring system of one or more saturated or unsaturated rings of which at least one ring contains a nitrogen and optionally one or more further heteroatoms selected from the group consisting of nitrogen, oxygen and/or sulfur in the ring; preferably is a heterocyclic ring system of one or two saturated or unsaturated rings of which at least one ring contains a nitrogen and optionally one or more further heteroatoms selected from the group consisting of nitrogen, oxygen and/or sulfur in the ring, more preferably is selected from azetidine, azepane, oxazepam, pyrrolidine, imidazole, oxadiazole, tetrazole, azetidine, pyridine, pyrimidine, piperidine, piperazine, benzimidazole, indazole, benzothiazole, benzodiazole, morpholine, indoline, triazole, isoxazole, pyrazole, pyrrole, pyrazine, pyrrolo[2,3-*b*]pyridine, quinoline, quinolone, isoquinoline, tetrahydrothienopyridine, phthalazine, benzo-1,2,5-thiadiazole, indole, benzotriazole, benzoxazole oxopyrrolidine, carbazole, thiazole, 3,9-diazaspiro[5.5]undecane, 2,8-diazaspiro[4.5]decane, 2,7-diazaspiro[3.5]nonane, 2,7-diazaspiro[4.4]nonane or octahydropyrrolo[3,4-*c*]pyrrole.

In connection with aromatic heterocyclyls (heteroaryls), non-aromatic heterocyclyls, aryls and cycloalkyls, when a ring system falls within two or more of the above cycle definitions simultaneously, then the ring system is defined first as an aromatic heterocyclyl (heteroaryl) if at least one aromatic ring contains a heteroatom. If no aromatic ring contains a heteroatom, then the ring system is defined as a non-aromatic heterocyclyl if at least one non-aromatic ring contains a heteroatom. If no non-aromatic ring contains a heteroatom, then the ring system is defined as an aryl if it contains at least one aryl cycle. If no aryl is present, then the ring system is defined as a cycloalkyl if at least one non-aromatic cyclic hydrocarbon is present.

"Heterocycloalkyl" as referred to in the present invention, are understood as meaning saturated and unsaturated (but not aromatic), generally 5 or 6 membered cyclic hydrocarbons which can optionally be unsubstituted, mono- or polysubstituted and which have at least one heteroatom in their structure selected from N, O or S. Examples for heterocycloalkyl radical preferably include but are not restricted to pyrroline, pyrrolidine, pyrazoline, aziridine, azetidine, tetrahydropyrrole, oxirane, oxetane, dioxetane, tetrahydropyrane, tetrahydrofurane, dioxane, dioxolane, oxazolidine, piperidine, piperazine, morpholine, azepane or diazepane. Heterocycloalkyl radicals, as defined in the present invention, are optionally mono- or polysubstituted by substitutents independently selected from a halogen atom, branched or unbranched C₁₋₆-alkyl, branched or unbranched C₁₋₆-alkoxy, C₁₋₆-haloalkoxy, C₁₋₆-haloalkyl, trihaloalkyl or a hydroxyl group. More preferably heterocycloalkyl in the context of the present invention are 5 or 6-membered ring systems optionally at least monosubstituted.

"Aryl" as referred to in the present invention, is understood as meaning ring systems with at least one aromatic ring but without heteroatoms even in only one of the rings. These aryl radicals may optionally be mono-or polysubstituted by substitutents independently selected from a halogen atom, -CN, branched or unbranched C₁₋₆-alkyl, branched or unbranched C₁₋₆-alkoxy, C₁₋₆₋haloalcoxy, C₁₋₆-haloalkyl, a heterocyclyl group and a hydroxyl group. Preferred examples of aryl radicals include but are not restricted to phenyl, naphthyl, fluoranthenyl, fluorenyl, tetralinyl, indanyl or anthracenyl radicals, which may optionally be mono- or polysubstituted, if not defined otherwise. More preferably aryl in the context of the present invention is a 6-membered ring system optionally at least mono or polysubstituted.

"Heteroaryl" as referred to in the present invention, is understood as meaning heterocyclic ring systems which have at least one aromatic ring and contain one or more heteroatoms from the group consisting of N, O or S and may optionally be mono-or polysubstituted by substituents independently selected from a halogen atom, branched or unbranched C₁₋₆-alkyl, branched or unbranched C₁₋₆-alkoxy, C₁₋₆-haloalkoxy, C₁₋₆-haloalkyl trihaloalkyl or a hydroxyl group. Preferred examples of heteroaryls include but are not restricted to furan, benzofuran, pyrrole, pyridine, pyrimidine, pyridazine, pyrazine, quinoline, isoquinoline, phthalazine, triazole, pyrazole, isoxazole, indole, benzotriazole, benzodioxolane, benzodioxane, benzimidazole, carbazole and quinazoline. More preferably heteroaryl in the context of the present invention are 5 or 6-membered ring systems optionally at least monosubstituted.

The term "condensed" according to the present invention means that a ring or ring-system is attached to another ring or ring-system, whereby the terms "annulated" or "annelated" are also used by those skilled in the art to designate this kind of attachment.

The term "ring system" according to the present invention refers to a system consisting of at least one ring of connected atoms but including also systems in which two or more rings of connected atoms are joined with "joined" meaning that the respective rings are sharing one (like a spiro structure), two or more atoms being a member or members of both joined rings. The "ring system" thus defined comprises saturated, unsaturated or aromatic carbocyclic rings which contain optionally at least one heteroatom as ring member and which are optionally at least mono-substituted and may be joined to other carbocyclic ring systems such as aryl radicals, heteroaryl radicals, cycloalkyl radicals etc.

The terms "condensed", "annulated" or "annelated" are also used by those skilled in the art to designate this kind of join.

A "leaving group" is a group that in a heterolytic bond cleavage keeps the electron pair of the bond. Suitable leaving groups are well known in the art and include Cl, Br, I and - O-SO₂R¹⁴, wherein R¹⁴ is F, C₁₋₄-alkyl, C₁₋₄-haloalkyl, or optionally substituted phenyl. The preferred leaving groups are Cl, Br, I, tosylate, mesylate, triflate, nonaflate and fluorosulphonate.

"Protecting group" is a group that is chemically introduced into a molecule to avoid that a certain functional group from that molecule undesirably reacts in a subsequent reaction. Protecting groups are used, among others, to obtain chemoselectivity in chemical reactions. The preferred protecting group in the context of the invention are Boc (tert-butoxycarbonyl) or Teoc (2-(trimethylsilyl)ethoxycarbonyl).

The term "salt" is to be understood as meaning any form of the active compound according to the invention in which this assumes an ionic form or is charged and is coupled with a counter-ion (a cation or anion). The definition particularly includes physiologically acceptable salts, this term must be understood as equivalent to "pharmaceutically acceptable salts".

The term "pharmaceutically acceptable salts" in the context of this invention means any salt that is tolerated physiologically (normally meaning that it is not toxic, particularly as a result of the counter-ion) when used in an appropriate manner for a treatment, particularly applied or used in humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with:hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid. In addition, the pharmaceutically acceptable salts may be formed with a physiologically tolerated cation, preferably inorganic, particularly when used on humans and/or mammals. Salts with alkali and alkali earth metals are particularly preferred, as well as those formed with ammonium cations (NH₄⁺).Preferred salts are those formed with (mono) or (di)sodium, (mono) or (di)potassium, magnesium or calcium.These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals.

The compounds of the invention may be present in crystalline form or in amorphous form.

Any compound that is a solvate of a compound according to formula (I) defined above is understood to be also covered by the scope of the invention. Methods of solvation are generally known within the art. Suitable solvates are pharmaceutically acceptable solvates. The term "solvate" is to be understood as meaning any form of the active compound according to the invention in which this compound has attached to it via noncovalent binding another molecule (most likely a polar solvent) especially including hydrates and alcoholates, like methanolate or ethanolate.

The term "co-crystal" is to be understood as a crystalline material comprising a specific active compound with at least one additional component, usually a co-crystal former, and of which at least two of the constituents are held together by weak interactions. Weak interaction is being defined as an interaction which is neither ionic nor covalent and includes for example: hydrogen bonds, van der Waals forces, and π-π interactions.

The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the compounds of the invention: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (april 2002).

Any compound that is a prodrug of a compound of general formula (I) is within the scope of the invention. Particularly favored prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Any compound that is a *N*-oxide of a compound according to the invention like a compound according to formula (I) defined above is understood to be also covered by the scope of the invention.

The compounds of formula (I) as well as their salts or solvates are preferably in pharmaceutically acceptable or substantially pure form. By pharmaceutically acceptable pure form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts. This applies also to its solvates or prodrugs.

In a particular and preferred embodiment of the invention, **Rₐ** and **Rₐ** are a hydrogen atom.

In another particular and preferred embodiment of the invention, **R₁** is a hydrogen atom; or -O**R₁ₐ.**

Yet another particular and preferred embodiment of the invention is that where **R₁ₐ** is branched or unbranched C₁₋₆ alkyl radical.

In an even more prefered embodiment **R₁ₐ** is methyl.

Another particular and preferred embodiment of the invention is that where **R₂** is a hydrogen atom; a branched or unbranched C₁₋₆ alkyl radical; a -(CH₂)ₙ-OR₂ₐ; a -C(O)R_{2b}; or an optionally substituted -(CH₂)_{q}-C₃₋₉ heterocycloalkyl.

In an even more prefered embodiment **R₂** is a hydrogen atom; preferably methyl or ethyl; a -(CH₂)ₙ-OR₂ₐ; a -C(O)R_{2b}; or an optionally substituted -(CH₂)_{q}-C₃₋₉ heterocycloalkyl.

A still even more preferred embodiment is where **R₂ₐ** is a hydrogen atom; a branched or unbranched C₁₋₆ alkyl radical optionally, preferably an ethyl unsubstituted or substituted with -OH.

Again a still even more preferred embodiment is where is a C₃₋₉ heterocycloalkyl radical, preferably a tetrahydrofuranyl.

Still another particular and preferred embodiment of the invention is that where **R₂** represents one of the following moieties optionally substituted:

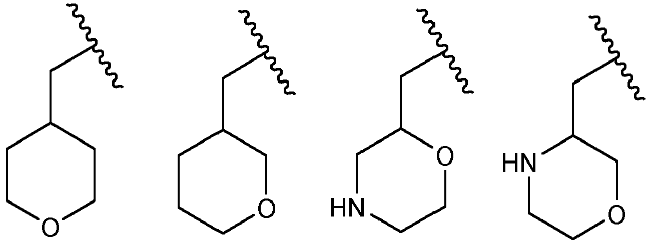

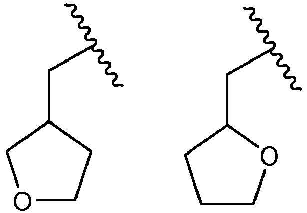

An additional particular and preferred embodiment of the invention is where **R₃** is an optionally substituted -(CH₂)ᵢ-C₃₋₉ cycloalkyl; an optionally substituted -(CH₂)ⱼ-C₃₋₉ heterocycloalkyl containing at least one heteroatom selected from N, O or S

In an even more preferred embodiment **R₃** represents one of the following moieties:

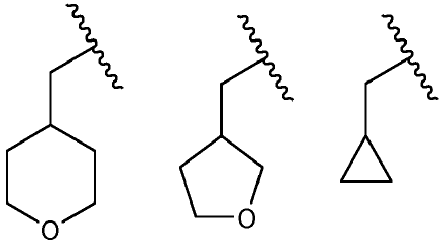

A further particular and preferred embodiment of the invention comprises a compound of formula (I): 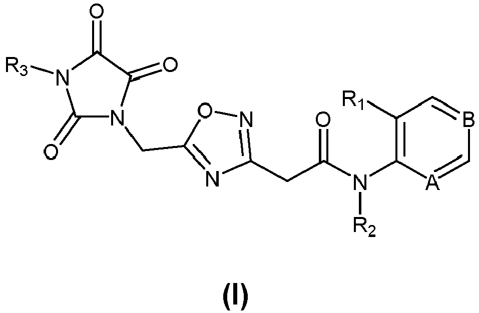 wherein:
**A** represents -CRₐ- or -N-;
**B** represents -CR_{b}- or -N-;
   **Rₐ** and **R_{b}** represent a hydrogen atom;
**R₁** is a hydrogen atom; or -O**R₁ₐ**;
   **R₁ₐ** is a branched or unbranched C₁₋₆ alkyl radical;
**R₂** is a hydrogen atom; a branched or unbranched C₁₋₆alkyl radical optionally substituted; a -(CH₂)ₙ-OR₂ₐ; a -C(O)R_{2b}; an optionally substituted -(CH₂)_{q}-C₃₋₉ heterocycloalkyl containing at least one heteroatom selected from N, O or S; all of the radicals optionally substituted being substituted by at least one substituent selected from a branched or unbranched C₁₋₆ alkyl radical, =O, -OR_{2'};
   **R_{2'}, R_{2"}**, and **R_{2‴}** are independently selected from a hydrogen atom or branched or unbranched C₁₋₆ alkyl radical;
**R₂ₐ** represent a hydrogen atom; a branched or unbranched C₁₋₆ alkyl radical optionally substituted with at least an R_{2a'};
   **R_{2a'}** is a -OH;
**R_{2b}** represents a C₃₋₉ heterocycloalkyl radical optionally substituted by at least one -R_{2b"};
   **R**_{**2b**"} is selected from a hydrogen atom or branched or unbranched C₁₋₆ alkyl radical;
**R₃** is an-(CH₂)ᵢ-C₃₋₉ cycloalkyl; an-(CH₂)ⱼ-C₃₋₉ heterocycloalkyl containing at least one heteroatom selected from N, O or S;
**n** is 0, 1, 2 or 3;
**q** is 0, 1, 2 or 3;
**i** is 0, 1, 2 or 3;
**j** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

A stil more particular and preferred embodiment of the invention comprises a compound of formula (I): 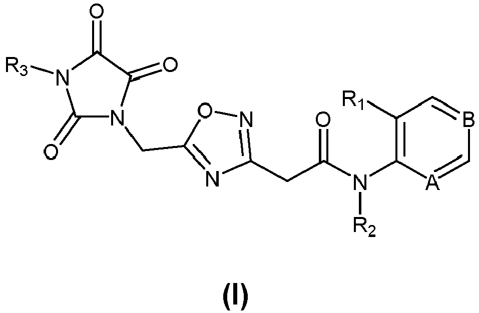 wherein:
**A** represents -CRₐ- or -N-;
**B** represents -CR_{b}- or -N-;
   **Rₐ** and **R_{b}** represent a hydrogen atom;
**R₁** is a hydrogen atom; or -O**R₁ₐ**;
   **R₁ₐ** is a methyl radical;
**R₂** is a hydrogen atom; methyl; ethyl; a -(CH₂)ₙ-OR₂ₐ; a -C(O)R_{2b} or one of the following moieties: 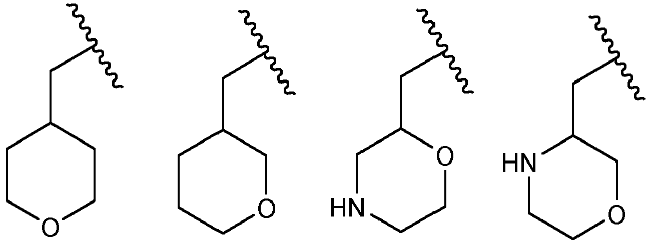 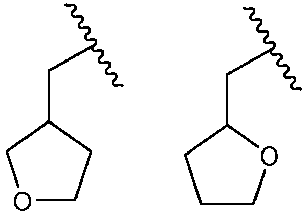
optionally substituted substituted by at least one substituent selected from a branched or unbranched C₁₋₆ alkyl radical, =O, -OR_{2'}; **R_{2'}** being selected from a hydrogen atom or branched or unbranched C₁₋₆ alkyl radical;
**R₂ₐ** represent a hydrogen atom; an ethyl unsubstituted or substituted with -OH;
**R_{2b}** represents a tetrahydrofuranyl;
**R₃** represents one of the following moieties: 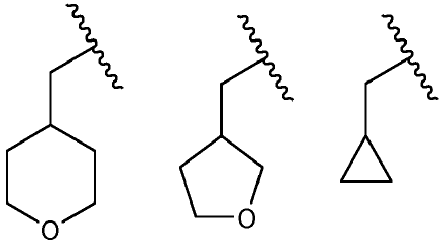
**n** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

The compounds of the present invention represented by the above described formula (I) may include enantiomers depending on the presence of chiral centers or isomers depending on the presence of double bonds (e.g. Z, E). The single stereoisomers, enantiomers or diastereoisomers and mixtures thereof fall within the scope of the present invention.

The preferred compounds of the invention are selected from:
**[1]** 2-(5-((3-(Cyclopropylmethyl)-2,4,5-trioxoimidazolidin-1-yl)methyl)-1,2,4-oxadiazol-3-yl)-N-(2-hydroxyethyl)-N-(2-methoxyphenyl)acetamide;
**[2]** 2-(5-((3-(Cyclopropylmethyl)-2,4,5-trioxoimidazolidin-1-yl)methyl)-1,2,4-oxadiazol-3-yl)-N-(3-methoxypyridin-4-yl)acetamide;
**[3]** 2-(5-((3-(Cyclopropylmethyl)-2,4,5-trioxoimidazolidin-1-yl)methyl)-1,2,4-oxadiazol-3-yl)-N-(pyridin-2-yl)acetamide;
**[4]** 2-(5-((3-(cyclopropylmethyl)-2,4,5-trioxoimidazolidin-1-yl)methyl)-1,2,4-oxadiazol-3-yl)-N-(3-hydroxypropyl)-N-(2-methoxyphenyl)acetamide;
**[5]** 2-(5-((3-(cyclopropylmethyl)-2,4,5-trioxoimidazolidin-1-yl)methyl)-1,2,4-oxadiazol-3-yl)-N-(2-(2-hydroxyethoxy)ethyl)-N-(2-methoxyphenyl)acetamide;
**[6]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-4-yl)methyl)acetamide;
**[7]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-3-yl)methyl)acetamide;
**[8]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide;
**[9]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-3-yl)methyl)acetamide;
**[10]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-2-yl)methyl)acetamide;
**[11]** N-(2-methoxyphenyl)-2-(5-((2,4,5-trioxo-3-((tetrahydro-2H-pyran-4-yl)methyl)imidazolidin-1-yl)methyl)-1,2,4-oxadiazol-3-yl)acetamide;
**[12]** N-(2-hydroxyethyl)-N-(2-methoxyphenyl)-2-(5-((2,4,5-trioxo-3-((tetrahydro-2H-pyran-4-yl)methyl)imidazolidin-1-yl)methyl)-1,2,4-oxadiazol-3-yl)acetamide;
**[13]** N-(2-methoxyphenyl)-2-(5-((2,4,5-trioxo-3-((tetrahydrofuran-3-yl)methyl)imidazolidin-1-yl)methyl)-1,2,4-oxadiazol-3-yl)acetamide;
**[14]** N-(2-hydroxyethyl)-N-(2-methoxyphenyl)-2-(5-((2,4,5-trioxo-3-((tetrahydrofuran-3-yl)methyl)imidazolidin-1-yl)methyl)-1,2,4-oxadiazol-3-yl)acetamide;
**[15]** (R)-2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide;
**[16]** (S)-2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide;
**[17]** 2-(5-((3-(cyclopropylmethyl)-2,4,5-trioxoimidazolidin-1-yl)methyl)-1,2,4-oxadiazol-3-yl)-N-((5,5-dimethyltetrahydrofuran-2-yl)methyl)-N-(2-methoxyphenyl)acetamide;
**[18]** 2-(5-((3-(cyclopropylmethyl)-2,4,5-trioxoimidazolidin-1-yl)methyl)-1,2,4-oxadiazol-3-yl)-N-(2-methoxyphenyl)-N-(((2S,5S)-5-methyltetrahydrofuran-2-yl)methyl)acetamide;
**[19]** N-(2-(5-((3-(cyclopropylmethyl)-2,4,5-trioxoimidazolidin-1-yl)methyl)-1,2,4-oxadiazol-3-yl)acetyl)-N-(2-methoxyphenyl)tetrahydrofuran-2-carboxamide;
**[20]** 2-(5-((3-(cyclopropylmethyl)-2,4,5-trioxoimidazolidin-1-yl)methyl)-1,2,4-oxadiazol-3-yl)-N-(2-methoxyphenyl)-N-((5-oxotetrahydrofuran-2-yl)methyl)acetamide;
**[21]** 2-(5-((3-(cyclopropylmethyl)-2,4,5-trioxoimidazolidin-1-yl)methyl)-1,2,4-oxadiazol-3-yl)-N-((4-hydroxytetrahydrofuran-2-yl)methyl)-N-(2-methoxyphenyl)acetamide;
**[22]** 2-(5-((3-(cyclopropylmethyl)-2,4,5-trioxoimidazolidin-1-yl)methyl)-1,2,4-oxadiazol-3-yl)-N-(2-methoxyphenyl)-N-(morpholin-2-ylmethyl)acetamide;
**[23]** 2-(5-((3-(cyclopropylmethyl)-2,4,5-trioxoimidazolidin-1-yl)methyl)-1,2,4-oxadiazol-3-yl)-N-(2-methoxyphenyl)-N-(morpholin-3-ylmethyl)acetamide;
**[24]** 2-(5-((3-(Cyclopropylmethyl)-2,4,5-trioxoimidazolidin-1-yl)methyl)-1,2,4-oxadiazol-3-yl)-*N*-ethyl-*N*-(2-methoxyphenyl)acetamide;
**[25]** 2-(5-((3-(Cyclopropylmethyl)-2,4,5-trioxoimidazolidin-1-yl)methyl)-1,2,4-oxadiazol-3-yl)-*N*-phenylacetamide;
[26] 2-(5-((3-(Cyclopropylmethyl)-2,4,5-trioxoimidazolidin-1-yl)methyl)-1,2,4-oxadiazol-3-yl)-*N*-(2-methoxyphenyl)-*N*-methylacetamide; or
[27] 2-(5-((3-(Cyclopropylmethyl)-2,4,5-trioxoimidazolidin-1-yl)methyl)-1,2,4-oxadiazol-3-yl)-*N*-(2-methoxyphenyl)acetamide;
or a pharmaceutical acceptable salt, stereoisomer, co-crystal, prodrug or solvate thereof.

In another aspect, the invention refers to the processes for obtaining the compounds of general formula (I). Several procedures have been developed for obtaining all the compounds of the invention, and the procedures will be explained below in methods A and B.

The obtained reaction products may, if desired, be purified by conventional methods, such as crystallization and chromatography. Where the processes described below for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

### METHOD A

Method A represents a first process for synthesizing compounds according to general formula (I): 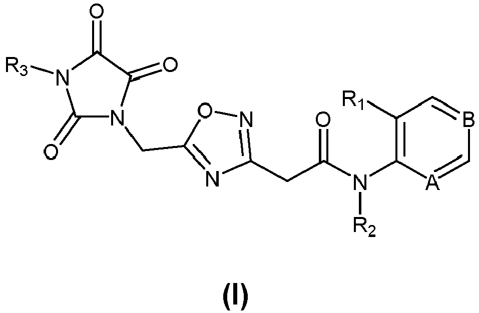
which comprises reacting a compound of formula (IX): 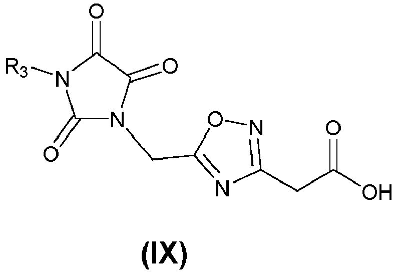
with a compound of formula **(X):** 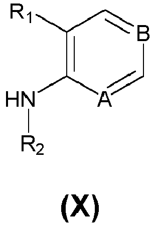
wherein **A, B, R₁**, **R₂** and **R₃** are as defined along the detailed description and the claims.

Scheme 1 below summarizes the synthetic routes of methods A. The reactions taken place in the different steps of scheme 1 (steps a to i) are representatively explained by means of particular embodiments in the examples.

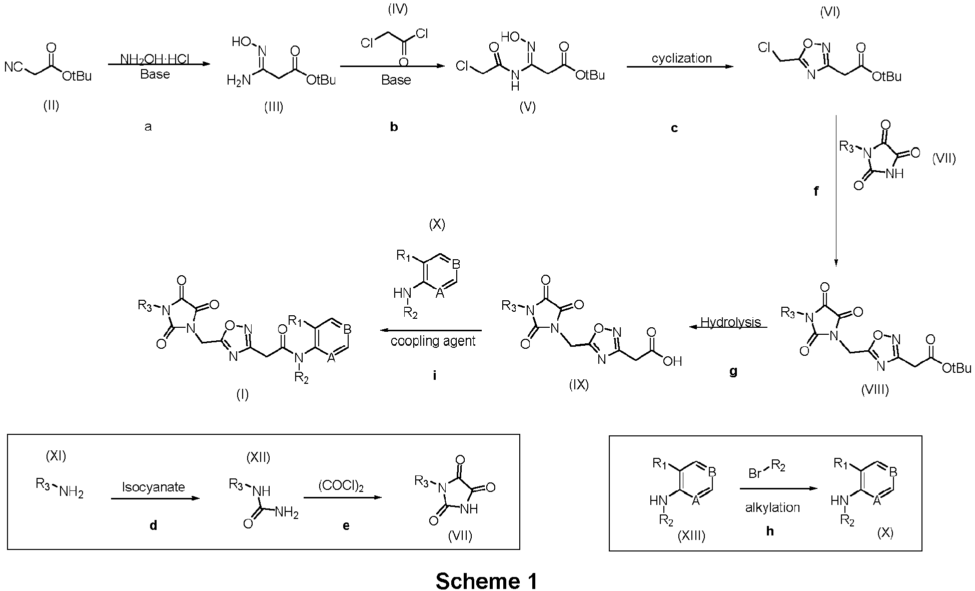

### METHOD B

Method B represents a second process for synthesizing compounds according to general formula (I).

Therefore, a process is described for the preparation of a compound of general formula (I): 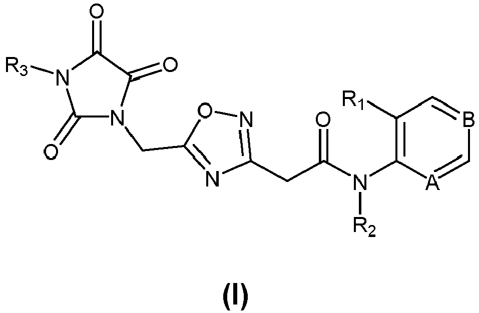
comprising the reaction between a compound of general formula **(XIX):** 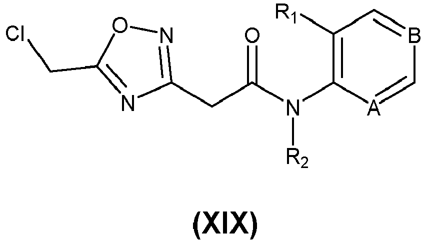
with a compound of formula (XIX): 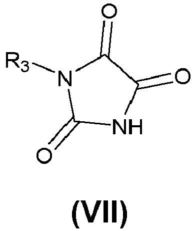
wherein **A, B, R₁**, **R₂** and **R₃** are as defined along the detailed description and the claims.

The reactions taken place in the different steps of scheme 2 (steps k to p) are representatively explained by means of particular embodiments in the examples.

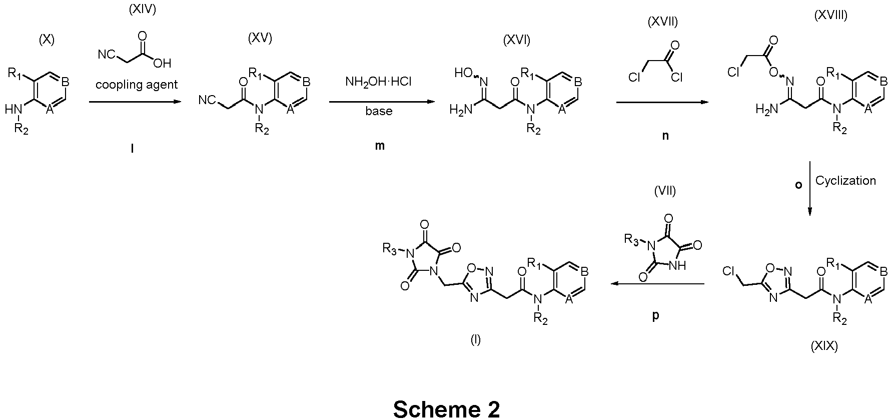

The compounds compounds of formula (II), (IV), (XI), (XIII), (XIV), (XVII) and (XVI) used in the methods disclosed above are commercially available or can be synthesized following common procedures described in the literature and exemplified in the synthesis of some intermediates.

Moreover, certain compounds of the present invention can also be obtained starting from other compounds of general formula (I) by appropriate conversion reactions of functional groups, in one or several steps, using well-known reactions in organic chemistry under standard experimental conditions.

In addition, a compound of general formula (I) that shows chirality can also be obtained by resolution of a racemic compound of general formula (I) either by chiral preparative HPLC or by crystallization of a diastereomeric salt or co-crystal. Alternatively, the resolution step can be carried out at a previous stage, using any suitable intermediate.

Turning to another aspect, the invention also relates to the therapeutic use of the compounds of general formula (I). As mentioned above, compounds of general formula (I) show a strong antibacterial activity against *Staphylococcus sp.,* especially to *Staphylococcus aureus.*

Therefore, compounds of general formula (I) are useful as medicaments and more particularly compounds of formula (I) are useful as antibiotic.

The compounds of formula (I) according to the invention are suitable for the treatment and/or prophylaxis of *Staphylococcus sp.* infections.

The compounds of general formula (I) are especially suited for the treatment of infections caused by *Staphylococcus aureus.*

A related aspect of the invention refers to the use of compounds of general formula (I) for the manufacture of a medicament for the treatment and/or prophylaxis of *Staphylococcus sp.* infections, more preferably infections caused by *Staphylococcus aureus.*

Another related aspect of the invention refers to a method for the treatment and/or prophylaxis of *Staphylococcus sp.* infections, more preferably infections caused by *Staphylococcus aureus* comprising the administration of a therapeutically effective amount of a compound of general formula (I) to a subject in need thereof.

Another aspect of the invention is a pharmaceutical composition, which comprises at least a compound of general formula (I) or a pharmaceutically acceptable salt, isomer, co-crystal, prodrug or solvate thereof, and at least a pharmaceutically acceptable carrier, additive, adjuvant or vehicle.

The pharmaceutical composition of the invention can be formulated as a medicament in different pharmaceutical forms comprising at least a compound binding to the sigma receptor and optionally at least one further active substance and/or optionally at least one auxiliary substance.

The auxiliary substances or additives can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical composition in accordance with the invention can be adapted to any form of administration, be it orally or parenterally, for example pulmonarily, nasally, rectally and/or intravenously.

Preferably, the composition is suitable for oral or parenteral administration, more preferably for oral, intravenous, intraperitoneal, intramuscular, subcutaneous, intrathekal, rectal, transdermal, transmucosal or nasal administration.

The composition of the invention can be formulated for oral administration in any form preferably selected from the group consisting of tablets, drageés, capsules, pills, chewing gums, powders, drops, gels, juices, syrups, solutions and suspensions. The composition of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.

Suitable preparations for parenteral applications are solutions, suspensions, reconstitutable dry preparations or sprays.

The compounds of the invention can be formulated as deposits in dissolved form or in patches, for percutaneous application.

Skin applications include ointments, gels, creams, lotions, suspensions or emulsions.

The preferred form of rectal application is by means of suppositories.

In a preferred embodiment, the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions, or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

The daily dosage for humans and animals may vary depending on factors that have their basis in the respective species or other factors, such as age, sex, weight or degree of illness and so forth. The daily dosage for humans may preferably be in the range from 1 to 2000, preferably 1 to 1500, more preferably 1 to 1000 milligrams of active substance to be administered during one or several intakes per day.

The following examples are merely illustrative of certain embodiments of the invention and cannot be considered as restricting it in any way.

### EXAMPLES

In the next examples the preparation of both intermediate compounds as well as compounds according to the invention are disclosed.

The following abbreviations are used:
anh: anhydrous
aq: aqueous
br s: broad singlet
C: Celsius
Cat: catalytic
CDMT: 2-chloro-4,6-dimethoxy-1,3,5-triazine
DIPEA: N-ethyl-N,N-diisopropylamine
DMAP: 4-Dimethylaminopyridine
DMF: N,N-dimethylformamide
EDCi: 1-(3-dimethylaminopropyl)-3-ethylcarbodiimide hydrochloride
Eq: equivalents
ESI: electrospray ionization
Et₂O: diethyl ether
EtOAc: ethyl acetate
EtOH: ethanol
Ex: example
g: gram
h: hour/s
HPLC: high-performance liquid chromatography
Hz: hertz
Int: intermediate
L: liter
m: meter, mili, multiplet
M: molar, molecular mass
m/z: mass-to-charge ratio
Me: methyl
MeCN: acetonitrile
MeOH: methanol
MS: mass spectrometry
min: minutes
NMM: 4-methylmorpholine (*N*-methylmorpholine)
NMR: nuclear magnetic resonance
Ret: retention
r.t.: room temperature
sat: saturated
THF: tetrahydrofuran
TMSNCO: (trimethylsilyl)isocyanate
UPLC: ultra-performance liquid chromatography

The following methods were used to determine the HPLC-MS spectra:
**A:** Column Kinetex C18 2.6 µm, 2.1x50 mm; temperature: 40 °C; flow rate: 0.50 mL/min; A: 50 mM ammonium formate buffer pH 4 with HCOOH, B: water; C: acetonitrile, gradient A:B:C: 0.3 min in 5:85:10 + from 5:85:10 to 5:10:85 in 1.7 min + 3 min in 5:10:85.
**B:** Column Kinetex C18 2.6 µm, 2.1x50 mm; temperature: 40 °C; flow rate: 0.50 mL/min; A: 50 mM ammonium formate buffer pH 4 with HCOOH, B: water; C: acetonitrile, gradient A:B:C: 0.3 min in 5:25:70 + from 5:25:70 to 5:0:95 in 1.7 min + 3 min in 5:0:95.
**C:** Column: SunFire C18 3.5 µm, 2.1 × 100 mm; temperature: 35 °C; flow rate: 0.30 mL/min; A: acetonitrile:/MeOH (1:1); B: Water; C: 100 mM ammonium acetate pH 7, gradient: A:B:C 5 min in 10:85:5 + from 10:85:5 to 95:0:5 in 15 min + 10 min in 95:0:5.
**D:** Column Kinetex C182.7 µm, 2.1x50 mm; temperature: 40 °C; flow rate: 0.40 mL/min; A: water, B: MeCN:MeOH (1:1); C: 100 mM ammonium acetate solution (pH 6.8), gradient A:B:C: 0.3 min in 85:10:5 + from 85:10:5 to 0:95:5 in 2 min + 2.7 min in 0:95:5.
**E:** Column Kinetex C18 2.6 µm, 2.1x50 mm; temperature: 40 °C; flow rate: 0.50 mL/min; A: water, B: MeCN; C: 50 mM ammonium acetate solution (pH 6.8), gradient A:B:C: 0.3 min in 85:10:5 + from 85:10:5 to 10:85:5 in 1.7 min + 3 min in 10:85:5.

The following methods were used to determine the UPLC-MS spectra:
**F:** Column Acquity BEH C18 1.7 µm 2.1 × 50 mm; temperature: 35 °C; flow rate: 0.50 mL/min; A: 50 mM ammonium formate buffer, pH 4 with HCOOH, B: water; C: acetonitrile, gradient A:B:C: 0.5 min in 5:85:10 + from 5:85:10 to 5:10:85 in 4.5 min + 4 min in 5:10:85.
**G:** Column Acquity BEH C18 1.7 µm, 2.1 × 100 mm; temperature: 35 °C; flow rate: 0.50 mL/min; A: 50 mM ammonium formate buffer, pH 4 with HCOOH, B: water; C: acetonitrile, gradient A:B:C: 0.5 min in 5:85:10 + from 5:85:10 to 5:10:85 in 4.5 min + 4 min in 5:10:85.
**H:** Column ZORBAX SB-C18 Rapid Resolution HD 1.8 µm, 2.1 × 50 mm; temperature: 35 °C; flow rate: 0.80 mL/min: A: 10 mM ammonium formate buffer, pH 3 with HCOOH, B: acetonitrile, gradient A:B: 0.2 min 95:5 + from 95:5 to 0:100 in 3.3 min + 0.5 min in 0:100.
**I**: Column ZORBAX SB-C18 Rapid Resolution HD 1.8 µm, 2.1 × 50 mm; temperature: 35 °C; flow rate: 0.80 mL/min: A: 10 mM ammonium formate buffer, pH 4 with HCOOH, B: acetonitrile, gradient A:B: 0.2 min 95:5 + from 95:5 to 0:100 in 3.3 min + 0.5 min in 0:100.

The following paragraps represent by way of example the preparation of certain intermediates and compounds according to formula (I) where the steps identified with letters correspond to those synthetic steps described in scheme 1.

### Example 1. 2-(5-((3-(Cyclopropylmethyl)-2,4,5-trioxoimidazolidin-1-yl)methyl)-1,2,4-oxadiazol-3-yl)-N-(2-hydroxyethyl)-N-(2-methoxyphenyl)acetamide

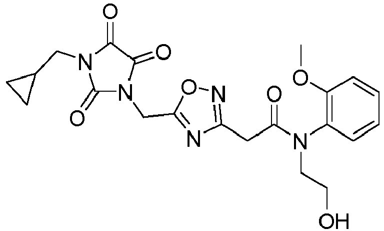

### a) tert-Butyl-3-amino-3-(hydroxyimino)propanoate

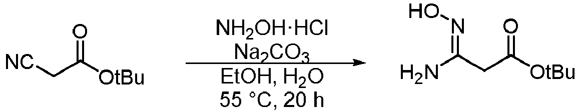

NH₂OH·HCl (5.7 g, 81.82 mmol) and Na₂CO₃ (8.7 g, 81.82 mmol) were added to a solution of *tert*-butyl 2-cyanoacetate (10.5 g, 74.38 mmol) in EtOH:water (4:1, 100 mL), and the suspension was stirred at 55 °C for 20 h. The reaction was allowed to reach room temperature, EtOH was concentrated off and the residue was diluted with EtOAc (100 mL) and washed with water (1x15 mL). The organic layer was dried over anhydrous Na₂SO₄, filtered and concentrated, affording 12.6 g of an orange solid. This solid was slurred with Et₂O (20 mL) and decanted off (x2) and dried, to afford *tert*-butyl-3-amino-3-(hydroxyimino)propanoate (10.03 g, pale orange solid, 77% yield).

ESI⁺-MS *m*/*z,* 175.1 (M+H).

¹H-NMR (CDCl₃, 300 MHz) δ ppm: 5.05 (brs, 2H), 3.08 (s, 2H), 1.45 (s, 9H).

### b) tert-Butyl 3-(2-chloroacetamido)-3-(hydroxyimino)propanoate

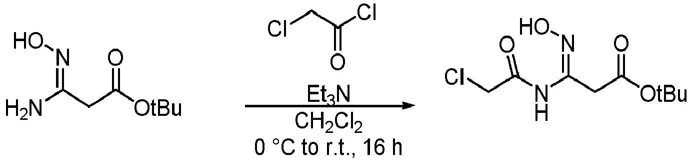

2-Chloroacetyl chloride (2.6 mL, 32.55 mmol) was added to a 0 °C cooled suspension of *tert*-butyl-3-amino-3-(hydroxyimino)propanoate (5.40 g, 31.00 mmol) and Et₃N (4.73 mL, 34.1 mmol) in CH₂Cl₂ (40 mL). The mixture was allowed to reach r.t. and allowed to react for 16 h. The reaction mixture was washed with water (2x30 mL), the organic layer was dried over Na₂SO₄ (anhydrous), filtered and concentrated. The crude was submitted to the next step without further purification (7.20 g, pale yellow solid, 93% yield).

### c) tert-Butyl 2-(5-(chloromethyl)-1,2,4-oxadiazol-3-yl)acetate

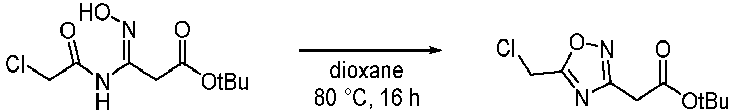

A solution of *tert*-butyl 3-(2-chloroacetamido)-3-(hydroxyimino)propanoate (7.15 g, 28.52 mmol) in dioxane (50 mL) was stirred at 80 °C for 16 h. Once full conversion was achieved, the solvent was concentrated off and the oil so obtained was dried, to afford *tert*-butyl 2-(5-(chloromethyl)-1,2,4-oxadiazol-3-yl)acetate (7.5 g, pale brown oil, >theoretical).

¹H-NMR (CDCl₃, 300 MHz) δ ppm: 4.68 (s, 2H), 3.69 (s, 2H), 1.45 (s, 9H).

### d) 1-(Cyclopropylmethyl)urea

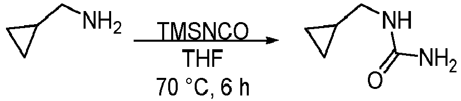

Cyclopropylmethanamine (4.00 mL, 46.12 mmol) was dropwise added to a solution of (trimethylsilyl)isocyanate (7.50 mL, 55.34 mmol) in THF (40 mL). The reaction mixture was warmed up to 70 °C and allowed to react for 6 h. Solvent was concentrated off, and the crude residue was slurred with hexanes (10 mL), filtered off and washed with hexanes (1x5 mL). The solid so obtained was dried, to afford 1-(cyclopropylmethyl)urea (3.44 g, white solid, 65% yield).

¹H-NMR (DMSO-d₆, 300 MHz) δ ppm: 5.95 (brs, 1H), 5.38 (brs, 2H), 2.83 (m, 2H), 0.85 (m, 1H), 0.36 (m, 2H), 0.10 (m, 2H).

### e) 1-(Cyclopropylmethyl)imidazolidine-2,4,5-trione

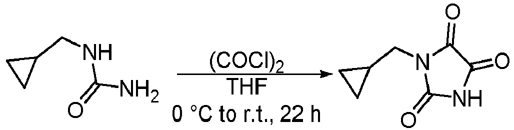

Oxalyl chloride (2.90 mL, 33.15 mmol) was dropwise added to a 0 °C cooled suspension of 1-(cyclopropylmethyl)urea (3.44 g, 30.14 mmol) in THF (35 mL). The mixture was allowed to reach r.t. and allowed to react for 22 h. The reaction mixture was poured into H₂O (20 mL) and extracted with EtOAc (3x60 mL). The combined organic layers were dried over Na₂SO₄ (anhydrous), filtered and concentrated. The crude was submitted to the next step without further purification (4.45 g, white solid, 88% yield).

¹H-NMR (CDCl₃, 300 MHz) δ ppm: 8.64 (brs, 1H), 3.51 (d, *J*=7.6 Hz, 2H), 1.18 (m, 1H), 0.57 (m, 2H), 0.37 (m, 2H).

### f) tert-Butyl 2-(5-((3-(cyclopropylmethyl)-2,4,5-trioxoimidazolidin-1-yl)methyl)-1,2,4-oxadiazol-3-yl)acetate

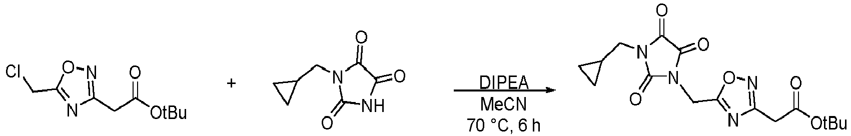

DIPEA (1.4 mL, 8.19 mmol) was added to a solution of *tert*-butyl 2-(5-(chloromethyl)-1,2,4-oxadiazol-3-yl)acetate (1.90 g, 8.19 mmol) and 1-(cyclopropylmethyl)imidazolidine-2,4,5-trione (1.06 g, 6.30 mmol) in MeCN (25 mL). The mixture was warmed up to 70 °C and stirred at this temperature for 6 h. Solvent was concentrated off and the crude was flash chromatographed on SiO₂ (15→40% EtOAc/hexanes) to furnish *tert-*butyl 2-(5-((3-(cyclopropylmethyl)-2,4,5-trioxoimidazolidin-1-yl)methyl)-1,2,4-oxadiazol-3-yl)acetate (1.8 g, pale yellow oil, 78% yield).

¹H-NMR (CDCl₃, 300 MHz) δ ppm: 5.08 (s, 2H), 3.71 (s, 2H), 3.56 (d, *J*=7.6 Hz, 2H), 1.44 (s, 9H), 1.17 (m, 1H), 0.58 (m, 2H), 0.39 (m, 2H).

### g) 2-(5-((3-(Cyclopropylmethyl)-2,4,5-trioxoimidazolidin-1-yl)methyl)-1,2,4-oxadiazol-3-yl)acetic acid

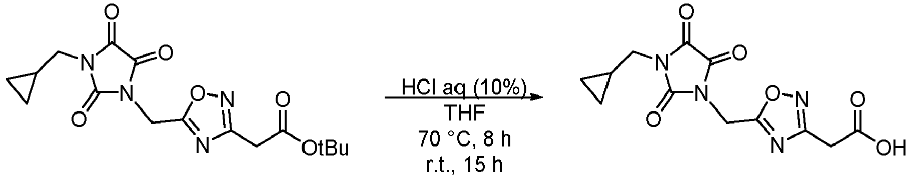

HCl (10% aqueous solution, 20 mL) was added to a solution of *tert*-butyl 2-(5-((3-(cyclopropylmethyl)-2,4,5-trioxoimidazolidin-1-yl)methyl)-1,2,4-oxadiazol-3-yl)acetate (1.80 g, 4.94 mmol) in THF (20 mL). The mixture was warmed up to 70 °C and stirred at this temperature for 8 h. The reaction mixture was allowed to reach r.t. and stirred at this temperature for 15 h. Solvent was concentrated off and the crude was extracted with CH₂Cl₂ (3×20 mL). The combined organic layers were dried over Na₂SO₄ (anhydrous), filtered and concentrated. The crude residue was slurred with hexanes (15 mL), filtered, eluted with hexanes (3x10 mL) and with Et₂O:hexanes (1:1, 1x8 mL) and dried, to afford the 2-(5-((3-(cyclopropylmethyl)-2,4,5-trioxoimidazolidin-1-yl)methyl)-1,2,4-oxadiazol-3-yl)acetic acid (1.27 g, white solid, 83% yield).

¹H-NMR (DMSO-d₆, 300 MHz) δ ppm: 12.85 (brs, 1H), 5.09 (s, 2H), 3.82 (s, 2H), 3.40 (d, *J*=6.8 Hz, 2H), 1.07 (m, 1H), 0.47 (m, 2H), 0.30 (m, 2H).

ESI⁺-MS *m*/*z,* 309.1 (M+H).

### h) N-(2-((tert-Butyldimethylsilyl)oxy)ethyl)-2-methoxyaniline

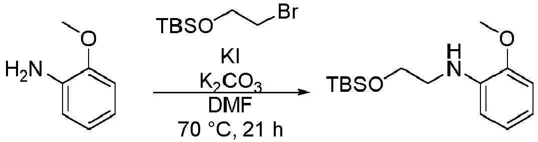

KI (444 mg, 2.68 mmol) and K₂CO₃ (4.44 g, 32.15 mmol) were added to a solution of o-anisidine (3.0 mL, 26.79 mmol) and (2-bromoethoxy)-*tert*-butyldimethylsilane (6.3 mL, 29.46 mmol) in DMF (50 mL). The mixture was warmed up to 70 °C and stirred at this temperature for 21 h. The mixture was allowed to reach r.t. and solvent was concentrated off. The residue was suspended in EtOAc (60 mL), washed with brine (3x80 mL) and with water (1x50 mL). The organic layer was dried over anhydrous Na₂SO₄, filtered and concentrated, affording 8.50 g of an orange oil. This crude was flash chromatographed on SiO₂ (1→5% EtOAc/hexanes) to furnish *N*-(2-((*tert*-butyldimethylsilyl)oxy)ethyl)-2-methoxyaniline (5.84 g, pale yellow solid, 77% yield).

¹H-NMR (CDCl₃, 300 MHz) δ ppm: 6.87 (m, 1H), 6.76 (m, 1H), 6.64 (m, 2H), 3.87 (m, 2H), 3.84 (s, 3H), 3.24 (m, 2H), 0.92 (s, 9H), 0.07 (s, 6H).

ESI⁺-MS *m*/*z,* 282.1 (M+H).

### i) N-(2-((tert-Butyldimethylsilyl)oxy)ethyl)-2-(5-((3-(cyclopropylmethyl)-2,4,5-trioxoimidazolidin-1-yl)methyl)-1,2,4-oxadiazol-3-yl)-N-(2-methoxyphenyl)acetamide

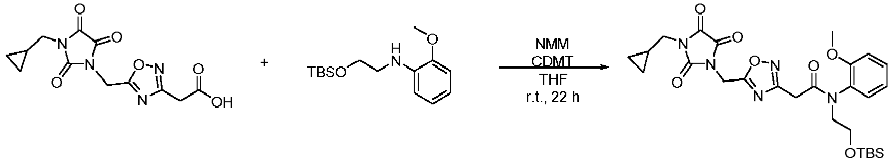

To a solution of 2-(5-((3-(cyclopropylmethyl)-2,4,5-trioxoimidazolidin-1-yl)methyl)-1,2,4-oxadiazol-3-yl)acetic acid (515 mg, 1.67 mmol) and *N-*(2-((*tert*butyldimethylsilyl)oxy)ethyl)-2-methoxyaniline (611 mg, 2.17 mmol) in THF (18 mL), NMM (0.240 mL, 2.17 mmol) and CDMT (381 mg, 2.17 mmol) were added, and the solution was stirred at r.t. for 22 h. The reaction mixture was diluted with EtOAc (30 mL) and washed with brine (2x15 mL). The combined aqueous layers were extracted with EtOAc (1x20 mL). The combined organic layers were dried Na₂SO₄ (anhydrous), filtered and concentrated. The crude was flash chromatographed on SiO₂ (30→50% EtOAc/hexanes) to furnish the title compound (658 mg, colorless foam, 53% yield).

¹H-NMR (CDCl₃, 300 MHz) δ ppm: 7.36-7.22 (m, 2H), 6.96 (m, 2H), 5.04 (s, 2H), 3.96 (m, 2H), 3.82 (s, 2H), 3.65 (m, 2H), 3.56-3.45 (m, 5H), 1.16 (m, 1H), 0.83 (s, 9H), 0.57 (m, 2H), 0.38 (m, 2H), 0.006 (s, 3H), -0.004 (s, 3H).

ESI⁺-MS *m*/*z,* 572.3 (M+H).

### j) Title compound

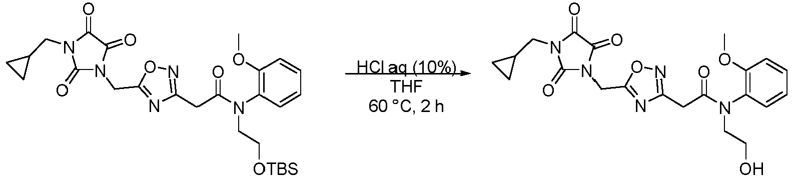

HCl (10% aqueous solution, 3.9 mL, 10.8 mmol) was added to a solution of *N-*(2-((*tert*butyldimethylsilyl)oxy)ethyl)-2-(5-((3-(cyclopropylmethyl)-2,4,5-trioxoimidazolidin-1-yl)methyl)-1,2,4-oxadiazol-3-yl)-N-(2-methoxyphenyl)acetamide (620 mg, 1.08 mmol) in THF (16 mL). The mixture was warmed up to 60 °C and stirred at this temperature until full conversion was achieved (2 h). The reaction mixture was allowed to reach r.t., poured into water (40 mL) and extracted with EtOAc (2x40 mL). The combined organic layers were washed with brine (1x40 mL), dried over Na₂SO₄ (anhydrous), filtered and concentrated. The crude residue was purified by flash chromatography on SiO₂ (40→60% acetone/hexanes) to afford 2-(5-((3-(cyclopropylmethyl)-2,4,5-trioxoimidazolidin-1-yl)methyl)-1,2,4-oxadiazol-3-yl)-*N*-(2-hydroxyethyl)-*N*-(2-methoxyphenyl)acetamide (466 mg, white solid, 94% yield).

¹H-NMR (CDCl₃, 300 MHz) δ ppm: 7.37 (t, *J*=7.5 Hz, 1H), 7.21 (d, *J*=6.5 Hz, 1H), 7.02 (m, 2H), 5.05 (s, 2H), 3.88 (m+s, 4H), 3.81-3.65 (m, 4H), 3.55 (s, 3H), 2.87 (m, 1H), 1.19 (m, 1H), 0.57 (m, 2H), 0.38 (m, 2H).

ESI⁺-MS *m*/*z,* 458.3 (M+H).

This method was used for the preparation of examples 2-23 using suitable starting materials:

| **Ex** | **Structure** | **Chemical name** | **MS (M+H)** | **Ret (min)** | **Method LC-MS** |
|---|---|---|---|---|---|
| 1 | 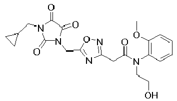 | 2-(5-((3-(Cyclopropylmethyl)-2,4,5-trioxoimidazolidin-1-yl)methyl)-1,2,4-oxadiazol-3-yl)-N-(2-hydroxyethyl)-N-(2-methoxyphenyl)acetamide | 458.3 | 3.300 | **F** |
| 2 | 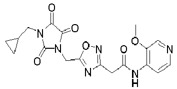 | 2-(5-((3-(Cyclopropylmethyl)-2,4,5-trioxoimidazolidin-1-yl)methyl)-1,2,4-oxadiazol-3-yl)-N-(3-methoxypyridin-4-yl)acetamide | 415.4 | 2.468 | **F** |
| 3 | 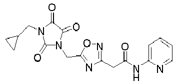 | 2-(5-((3-(Cyclopropylmethyl)-2,4,5-trioxoimidazolidin-1-yl)methyl)-1,2,4-oxadiazol-3-yl)-N-(pyridin-2-yl)acetamide | 385.3 | 3.075 | **F** |
| 4 | 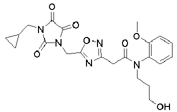 | 2-(5-((3-(cyclopropylmethyl)-2,4,5-trioxoimidazolidin-1-yl)methyl)-1,2,4-oxadiazol-3-yl)-N-(3-hydroxypropyl)-N-(2-methoxyphenyl)acetamide | 472.3 | 2.32 | **H** |
| 5 | 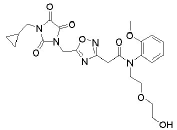 | 2-(5-((3-(cyclopropylmethyl)-2,4,5-trioxoimidazolidin-1-yl)methyl)-1,2,4-oxadiazol-3-yl)-N-(2-(2-hydroxyethoxy)ethyl)-N-(2-methoxyphenyl)acetamide | 502.3 | 2.28 | **I** |
| 6 | 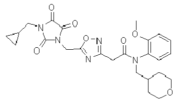 | 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-4-yl)methyl)acetamide | 512.4 | 2.61 | **I** |
| 7 | 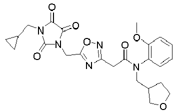 | 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-3-yl)methyl)acetamide | 498.4 | 2.55 | **I** |
| 8 | 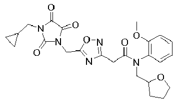 | 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide | 498.5 | 2.63 | **I** |
| 9 | 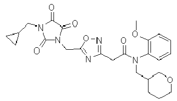 | 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-3-yl)methyl)acetamide | 512.5 | 2.68 | **I** |
| 10 | 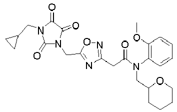 | 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-2-yl)methyl)acetamide | 512.4 | 2.74 | **I** |
| 11 | 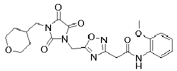 | N-(2-methoxyphenyl)-2-(5-((2,4,5-trioxo-3-((tetrahydro-2H-pyran-4-yl)methyl)imidazolidin-1-yl)methyl)-1,2,4-oxadiazol-3-yl)acetamide | 458.4 | 2.29 | **I** |
| 12 | 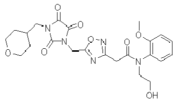 | N-(2-hydroxyethyl)-N-(2-methoxyphenyl)-2-(5-((2,4,5-trioxo-3-((tetrahydro-2H-pyran-4-yl)methyl)imidazolidin-1-yl)methyl)-1,2,4-oxadiazol-3-yl)acetamide | 502.4 | 2.08 | **I** |
| 13 | 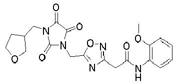 | N-(2-methoxyphenyl)-2-(5-((2,4,5-trioxo-3-((tetrahydrofuran-3-yl)methyl)imidazolidin-1-yl)methyl)-1,2,4-oxadiazol-3-yl)acetamide | 444.3 | 2.21 | **I** |
| 14 | 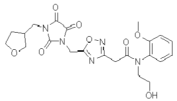 | N-(2-hydroxyethyl)-N-(2-methoxyphenyl)-2-(5-((2,4,5-trioxo-3-((tetrahydrofuran-3-yl)methyl)imidazolidin-1-yl)methyl)-1,2,4-oxadiazol-3-yl)acetamide | 488.4 | 2.01 | **I** |
| 15 | 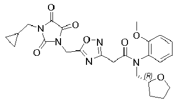 | (R)-2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide | | | |
| 16 | 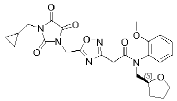 | (S)-2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide | | | |
| 17 | 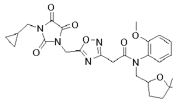 | 2-(5-((3-(cyclopropylmethyl)-2,4,5-trioxoimidazolidin-1-yl)methyl)-1,2,4-oxadiazol-3-yl)-N-((5,5-dimethyltetrahydrofuran-2-yl)methyl)-N-(2-methoxyphenyl)acetamide | | | |
| 18 | 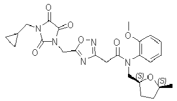 | 2-(5-((3-(cyclopropylmethyl)-2,4,5-trioxoimidazolidin-1-yl)methyl)-1,2,4-oxadiazol-3-yl)-N-(2-methoxyphenyl)-N-(((2S,5S)-5-methyltetrahydrofuran-2-yl)methyl)acetamide | | | |
| 19 | 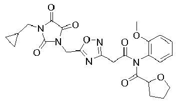 | N-(2-(5-((3-(cyclopropylmethyl)-2,4,5-trioxoimidazolidin-1-yl)methyl)-1,2,4-oxadiazol-3-yl)acetyl)-N-(2-methoxyphenyl)tetrahydrofura n-2-carboxamide | | | |
| 20 | 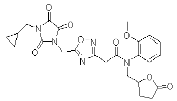 | 2-(5-((3-(cyclopropylmethyl)-2,4,5-trioxoimidazolidin-1-yl)methyl)-1,2,4-oxadiazol-3-yl)-N-(2-methoxyphenyl)-N-((5-oxotetrahydrofuran-2-yl)methyl)acetamide | | | |
| 21 | 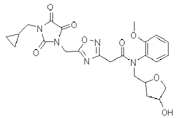 | 2-(5-((3-(cyclopropylmethyl)-2,4,5-trioxoimidazolidin-1-yl)methyl)-1,2,4-oxadiazol-3-yl)-N-((4-hydroxytetrahydrofuran-2-yl)methyl)-N-(2-methoxyphenyl)acetamide | | | |
| 22 | 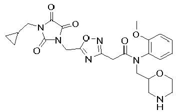 | 2-(5-((3-(cyclopropylmethyl)-2,4,5-trioxoimidazolidin-1-yl)methyl)-1,2,4-oxadiazol-3-yl)-N-(2-methoxyphenyl)-N-(morpholin-2-ylmethyl)acetamide | | | |
| 23 | 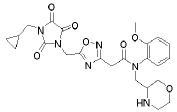 | 2-(5-((3-(cyclopropylmethyl)-2,4,5-trioxoimidazolidin-1-yl)methyl)-1,2,4-oxadiazol-3-yl)-N-(2-methoxyphenyl)-N-(morpholin-3-ylmethyl)acetamide | | | |

The following paragraps represent by way of example the preparation of certain intermediates and compounds according to formula (I) where the steps identified with letters correspond to those synthetic steps described in scheme 2.

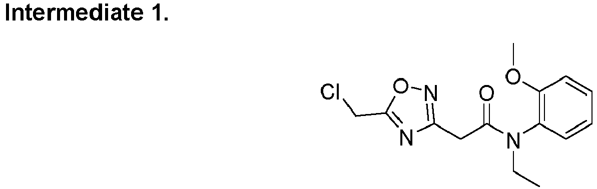

### k) N-Ethyl-2-methoxyaniline

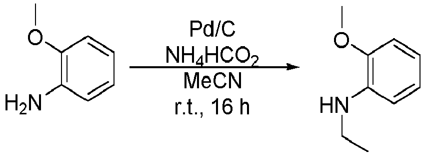

A solution of ammonium formate (6.00 g, 95.15 mmol, 11.7 eq) in water (10 mL) was added to a suspension of Pd/C 10% (430 mg, 0.406 mmol, 0.05 eq) and o-anisidine (1.09 mL, 8.12 mmol, 1 eq) in MeCN (40 mL). The resulting mixture was stirred at r.t. for 16 h. The suspension was filtered through a pad of celite, eluting with MeOH (50 mL). The solvent was concentrated off, the residue was dissolved in CH₂Cl₂ (20 mL), dried over Na₂SO₄ (anhydrous), filtered and concentrated. The crude was submitted to the next step without further purification (1.67 g, red oil, yield >theoretical).

¹H-NMR (CDCl₃, 300 MHz) δ ppm: 6.88 (t, *J=* 7.6 Hz, 1H), 6.77 (d, *J=* 7.9 Hz, 1H), 6.64 (m, 2H), 3.85 (s, 3H), 3.18 (c, *J=* 7.2 Hz, 3H), 1.30 (t, *J=* 7.2 Hz, 3H).

ESI⁺-MS *m*/*z,* 152.0 (M+H).

### l) 2-Cyano-N-ethyl-N-(2-methoxyphenyl)acetamide

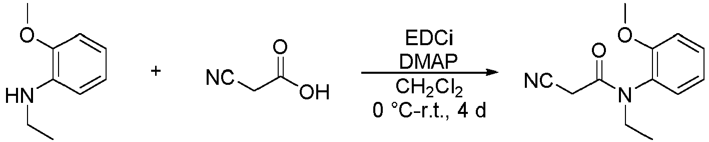

EDCi (1.71 g, 8.93 mmol, 1.1 eq) and DMAP (99 mg, 0.81 mmol, 0.1 eq) were added to a cooled (0 °C) suspension of cyanoacetic (760 mg, 8.93 mmol, 1.1 eq) in CH₂Cl₂ (10 mL). A solution of N-ethyl-2-methoxyaniline (8.12 mmol, 1.0 eq) in CH₂Cl₂ (12 mL) was added, and the reaction mixture was allowed to reach r.t. and stirred at r.t. for 4 days. The reaction mixture was diluted with water (20 mL) and extracted with CH₂Cl₂ (2x20 mL). The combined organic layers were dried Na₂SO₄ (anhydrous), filtered and concentrated. The crude was flash chromatographed on SiO₂ (5→23% EtOAc/hexanes) to furnish 2-cyano-*N*-ethyl-*N*-(2-methoxyphenyl)acetamide (1.05 g, brown solid, 59% yield).

¹H-NMR (CDCl₃, 300 MHz) δ ppm: 7.39 (t, *J=* 7.9, 1H), 7.16 (dd, *J* = 7.7, 1.8 Hz, 1H), 7.03 (m, 2H), 3.86 (s, 3H), 3.84-3.55 (m, 2H), 3.15 (s, 2H), 1.09 (t, *J=* 7.2 Hz, 3H). ESI⁺-MS *m*/*z,* 219.1 (M+H).

### m) 3-Amino-N-ethyl-3-(hydroxyimino)-N-(2-methoxyphenyl)propanamide

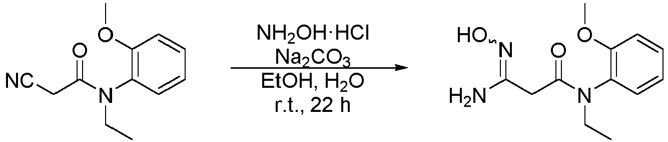

NH₂OH·HCl (401 mg, 5.77 mmol, 1.2 eq) and Na₂CO₃ (612 mg, 5.77 mmol, 1.2 eq) were added to a solution of 2-cyano-*N*-ethyl-*N*-(2-methoxyphenyl)acetamide (1.05 g, 4.81 mmol, 1.0 eq) in EtOH:water (5:1, 12 mL), and the suspension was stirred at r.t. for 22 h. EtOH was concentrated off and the residue was diluted with EtOAc (20 mL) and washed with water (1x15 mL). The organic layer was dried over anhydrous Na₂SO₄, filtered and concentrated, affording 1.21 g of a yellow oil. This oil was slurred with Et₂O (8 mL), filtered, washed with Et₂O (2x2 mL) and dried, to afford 3-amino-*N*-ethyl-3-(hydroxyimino)-*N*-(2-methoxyphenyl)propanamide (881 mg, white solid, 73% yield).

¹H-NMR (CDCl₃, 300 MHz) δ ppm: 7.34 (t, *J=* 7.9 Hz, 1H), 7.12 (dd, *J* = 7.7, 1.7 Hz, 1H), 7.06-6.92 (m, 2H), 5.23 (brs, 2H), 3.90-3.73 (m, 1H), 3.81 (s, 3H), 3.55 (m, 1H), 2.84 (d, *J=* 1.5 Hz, 2H), 1.08 (t, *J=* 7.2 Hz, 3H).

ESI⁺-MS *m*/*z,* 252.1 (M+H).

### n) 3-Amino-3-((2-chloroacetoxy)imino)-N-ethyl-N-(2-methoxyphenyl)propanamide

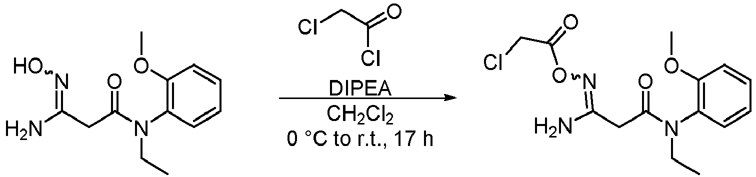

2-Chloroacetyl chloride (0.293 mL, 3.69 mmol, 1.05 eq) was added dropwise to a cooled solution (0 °C) of 3-amino-*N*-ethyl-3-(hydroxyimino)-*N*-(2-methoxyphenyl)propanamide (881 mg, 3.51 mmol, 1.0 eq) and DIPEA (0.71 mL, 4.04 mmol, 1.15 eq) in CH₂Cl₂ (10 mL). The reaction mixture was allowed to reach r.t. and stirred at this temperature for 17 h. The reaction mixture was diluted with water (15 mL) and extracted with CH₂Cl₂ (2×10 mL). The combined organic layers were dried over anhydrous Na₂SO₄, filtered and concentrated, to afford 3-amino-3-((2-chloroacetoxy)imino)-*N*-ethyl-*N*-(2-methoxyphenyl)propanamide. The crude was submitted to the next step without further purification (1.22 g, brown oil, yield >theoretical).

ESI⁺-MS *m*/*z,* 327.2, 329.1 (M+H).

### o) 2-(5-(Chloromethyl)-1,2,4-oxadiazol-3-yl)-N-ethyl-N-(2-methoxyphenyl)acetamide

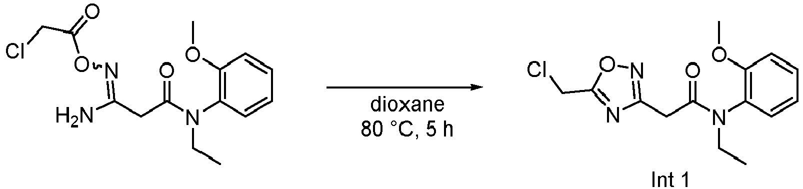

A solution of 3-amino-3-((2-chloroacetoxy)imino)-*N*-ethyl-*N*-(2-methoxyphenyl)propanamide (3.51 mmol, 1.0 eq) in dioxane (15 mL) was warmed up to 80 °C and stirred at this temperature for 5 h. Solvent was concentrated off, to afford 2-(5-(chloromethyl)-1,2,4-oxadiazol-3-yl)-*N*-ethyl-*N*-(2-methoxyphenyl)acetamide. The crude was submitted to the next step without further purification (1.68 g, brown oil, yield >theoretical).

This method was used for the preparation of intermediates 2-4 using suitable starting materials:

| **Int** | **Structure** | **Chemical name** | **MS (M+H)** | **Ret (min)** | **Method LC-MS** |
|---|---|---|---|---|---|
| Int 1 | 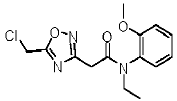 | 2-(5-(Chloromethyl)-1,2,4-oxadiazol-3-yl)-*N*-ethyl-*N-*(2-methoxyphenyl)acetamide | | | Not isolated |
| Int 2 | 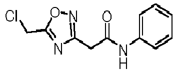 | 2-(5-(Chloromethyl)-1,2,4-oxadiazol-3-yl)-*N-*phenylacetamide | 250.2 (M-H) | 2.518 | **E** |
| Int 3 | 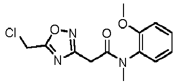 | 2-(5-(Chloromethyl)-1,2,4-oxadiazol-3-yl)-*N*-(2-methoxyphenyl)-*N-*methylacetamide | | | Not isolated |
| Int 4 | 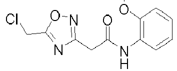 | 2-(5-(Chloromethyl)-1,2,4-oxadiazol-3-yl)-N-(2-methoxyphenyl)acetamide | 282.2 | 3.341 | **D** |

### Example 24. 2-(5-((3-(Cyclopropylmethyl)-2,4,5-trioxoimidazolidin-1-yl)methyl)-1,2,4-oxadiazol-3-yl)-N-ethyl-N-(2-methoxyphenyl)acetamide

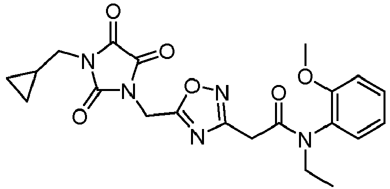

### p) 2-(5-((3-(Cyclopropylmethyl)-2,4,5-trioxoimidazolidin-1-yl)methyl)-1,2,4-oxadiazol-3-yl)-N-ethyl-N-(2-methoxyphenyl)acetamide

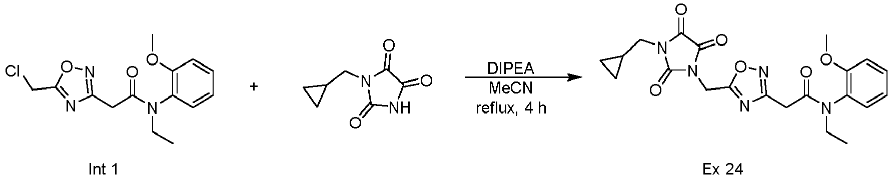

DIPEA (0.932 mL, 5.27 mmol, 1.5 eq) was added to a solution of 2-(5-(chloromethyl)-1,2,4-oxadiazol-3-yl)-*N*-ethyl-*N*-(2-methoxyphenyl)acetamide (3.51 mmol, 1.0 eq) and 1-(cyclopropylmethyl)imidazolidine-2,4,5-trione (708 mg, 4.21 mmol, 1.2 eq) in MeCN (20 mL). The mixture was refluxed until full conversion was achieved (4 h). The reaction mixture was allowed to reach r.t., solvent was concentrated off and the crude was flash chromatographed on SiO₂ (22→30% EtOAc/hexanes) to yield 2-(5-((3-(cyclopropylmethyl)-2,4,5-trioxoimidazolidin-1-yl)methyl)-1,2,4-oxadiazol-3-yl)-*N*-ethyl-*N*-(2-methoxyphenyl)acetamide (750 mg, pale yellow solid, 48% yield).

¹H-NMR (CDCl₃, 300 MHz) δ ppm: 7.35 (m, 1H), 7.16 (dd, *J* = 7.9, 1.7 Hz, 1H), 6.98 (m, 2H), 5.05 (s, 2H), 3.84 (s, 3H), 3.78 (q, *J=* 6.9 Hz, 1H), 3.68-3.41 (m, 3H), 1.64 (s, 2H), 1.31-1.13 (m, 1H), 1.07 (t, *J=* 7.2 Hz, 3H), 0.58 (t, *J=* 5.1 Hz, 2H), 0.39 (t, *J=* 5.1 Hz, 2H).

ESI⁺-MS *m*/*z,* 442.3.

This method was used for the preparation of examples 25-27 using suitable starting materials:

| **Ex** | **Structure** | **Chemical name** | **MS (M+H)** | **Ret (min)** | **Method (HPLC-MS) (UPLC-MS)** |
|---|---|---|---|---|---|
| 24 | 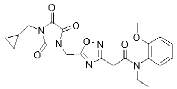 | 2-(5-((3-(Cyclopropylmethyl)-2,4,5-trioxoimidazolidin-1-yl)methyl)-1,2,4-oxadiazol-3-yl)-*N*-ethyl-*N*-(2-methoxyphenyl)acetamide | 442.3 | 3.947 | **F** |
| 25 | 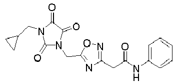 | 2-(5-((3-(Cyclopropylmethyl)-2,4,5-trioxoimidazolidin-1-yl)methyl)-1,2,4-oxadiazol-3-yl)-*N*-phenylacetamide | 384.2 | 3.505 | **G** |
| 26 | 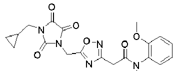 | 2-(5-((3-(Cyclopropylmethyl)-2,4,5-trioxoimidazolidin-1-yl)methyl)-1,2,4-oxadiazol-3-yl)-*N*-(2-methoxyphenyl)-*N-*methylacetamide | 428.3 | 3.675 | **G** |
| 27 | 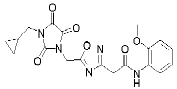 | 2-(5-((3-(Cyclopropylmethyl)-2,4,5-trioxoimidazolidin-1-yl)methyl)-1,2,4-oxadiazol-3-yl)-*N*-(2-methoxyphenyl)acetamide | 414.2 | 17.148 | **C** |

### Examples of biological activity

### Experimental procedure: MIC Test. Microdilution method

To evaluate the antimicrobial activity of the compounds, ATCC strains of Staphylococcus *aureus* were used. The minimal inhibitory concentrations (MICs) of the compounds were determined by microdilution assays, according to CLSI methodology.

### Microplate Set-up: Preparation of compounds

3mg of compound were weighted in a 4mL glass vials and were dissolved in properly volume of DMSO 100% (Reference: D2438-50, Sigma) to reach 12,8 mg/mL. (Cmax.).

### "Mother" plate

In 96-well plates U-form (Reference: 650161, Greiner Bio-One):
- Dispense 50µL of the assayed compounds prepared in DMSO 100% at 12,8 mg/mL in each well in column 1. (8 different compounds per plate, row 1 to row 8)
- Dispense 25µL of DMSO 100% in all wells in columns 2 to 12.
- With a multichannel pipette, aspire 25µL of column 1 and dispense in column 2. Mix the contents in column 2 several times and aspire 25uL. Dispense in column 3.
- Repeat the process until column 10. In column 10, aspire 25µL of the content and discard.
- The final mother plate map is (all values in mg/mL)

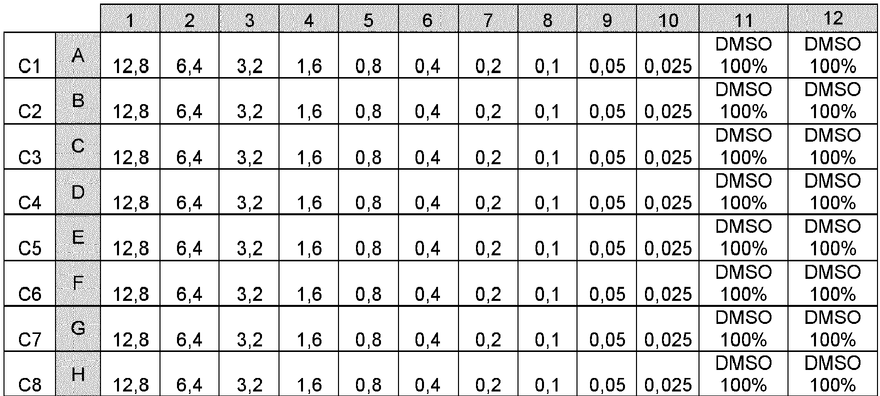

### "Daughter" plates:

In 96-well plates U-form:
- With a multichannel pipette (in dispensing mode), aspire a properly volume (consider the number of copies of the mother plate do you want to perform) of all wells in mother plate, to dispense **1µL/well** in daughter plates.

### NOTE:

Tecan instrument (FREEDOM EVO 100) was used to make the mother and daughter plates.

### MIC test: inoculation of bacteria

Bacterial suspension will be prepared and normalized to 0,5 McFarland turbidity standard (aprox. 10⁸ CFU/mL) using a physiological saline solution (sodium chloride 0,9%). Then, prepared a 1/1000 dilution (aprox. 10⁵ CFU/mL) of the suspension in appropriate media (CLSI Standard) and inoculate 99µL in each well in daughter plates (only columns 1 to 11) using a multidrop^{™} Combi Reagent Dispenser. In column 12, only 99µL of media will be dispense as a sterility control.

The final plate map is (all values in µg/mL):

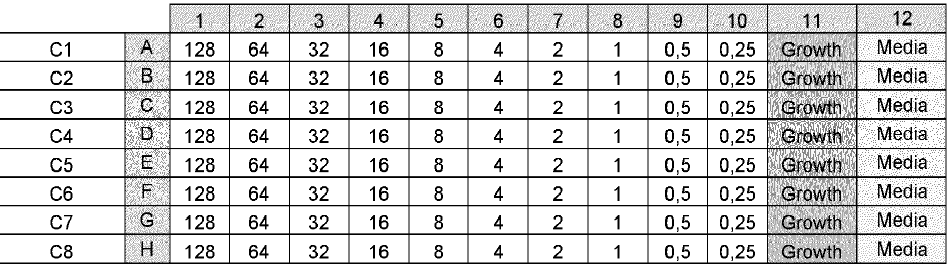

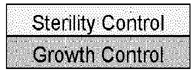

Plates will be incubated 18-20 h at 37°C. MIC values will be considered as the concentration of compounds at which the growth will be inhibited => ≥80%, compared to growth control.

### ACTIVITY RESULTS AGAINST STAPHYLOCOCCUS AUREUS:

| **Example** | **Activity (MIC)** in ug/mL |
|---|---|
| 1 | 4 |
| 4 | 16 |
| 5 | 32 |
| 24 | 8 |
| 26 | 4 |
| 27 | 4 |

## Claims

1. A compound of general formula (I): 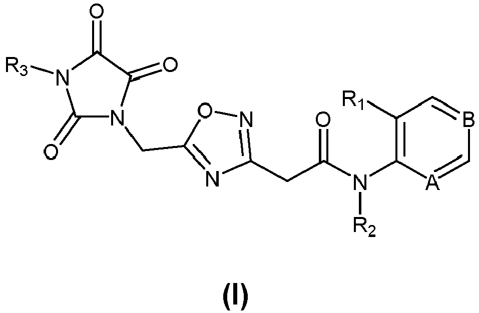 wherein:
**A** represents -CRₐ- or -N-;
**B** represents -CR_{b}- or -N-;
**Rₐ** and **R_{b}** independently represent a hydrogen atom; a halogen atom; -CN; a branched or unbranched C₁₋₆ alkyl radical; a branched or unbranched C₁₋₆ haloalkyl radical, an -O**R_{ab}** radical, or a C₃₋₉ cycloalkyl radical;
**R_{ab}** is a hydrogen atom or a branched or unbranched C₁₋₆ alkyl radical
**R₁** is a hydrogen atom; a halogen atom; a branched or unbranched C₁₋₆ alkyl radical or - O**R₁ₐ**;
**R₁ₐ** is a hydrogen atom or a branched or unbranched C₁₋₆ alkyl radical;
**R₂** is a hydrogen atom; a branched or unbranched C₁₋₆alkyl radical optionally substituted; a halogen atom; a -CN; a C₁₋₆ haloalkyl radical; a -(CH₂)ₙ-OR₂ₐ; a -C(O)R_{2b}; an optionally substituted -(CH₂)ₘ-C₃₋₉ cycloalkyl; an optionally substituted -(CH₂)_{q}-C₃₋₉ heterocycloalkyl containing at least one heteroatom selected from N, O or S; an optionally substituted -(CH₂)ᵣ-heteroaryl containing at least one heteroatom selected from N, O or S; an optionally substituted -(CH₂)ₛ-aryl; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, -NO₂,=O, -OR_{2'}, -NR₂,R_{2"}, NR_{2'}C(O)R_{2"}, -NR_{2'}S(O)₂R_{2"},-S(O)₂NR₂R_{2"}, -NR_{2'}C(O)NR_{2"}R_{2‴}, -SR_{2'}, -S(O)R_{2'}, S(O)₂R_{2'}, -C(O)OR_{2'}, -C(O)NR_{2'}R_{2"},-OCH₂CH₂OH, -NR_{2'}S(O)₂NR_{2"}R_{2‴}, -C(CH₃)₂OR_{2'};
**R_{2'}, R_{2"}**, and **R_{2‴}** are independently selected from a hydrogen atom or branched or unbranched C₁₋₆ alkyl radical;
**R₂ₐ** represent a hydrogen atom; a branched or unbranched C₁₋₆ alkyl radical optionally substituted with at least an R_{2a'}; a branched or unbranched C₁₋₆ haloalkyl radical, or a C₃₋₉ cycloalkyl radical;
**R_{2a'}** a hydrogen atom or -OH;
**R_{2b}** represent a hydrogen atom; a halogen atom; -CN; a branched or unbranched C₁₋₆ alkyl radical; a branched or unbranched C₁₋₆ haloalkyl radical, an -OR_{2b}' radical, or a C₃₋₉ heterocycloalkyl radical optionally substituted by at least one -R_{2b"};
**R_{2b'}** and **R_{2b"}** are independently selected from a hydrogen atom or branched or unbranched C₁₋₆ alkyl radical;
**R₃** is a hydrogen atom, an optionally substituted branched or unbranched C₁₋₆ alkyl; an optionally substituted -(CH₂)ᵢ-C₃₋₉ cycloalkyl; an optionally substituted -(CH₂)ⱼ-C₃₋₉ heterocycloalkyl containing at least one heteroatom selected from N, O or S; all of the radicals optionally substituted being substituted by at least one substituent selected from a hydrogen atom, halogen atom, a branched or unbranched C₁₋₆ alkyl radical, a C₁₋₆ haloalkyl radical, a C₁₋₆ haloalkoxy radical; -CN, -NO₂,=O, -OR_{3'}, -NR_{3'}R_{3"}, NR_{3'}C(O)R_{3"}, -NR_{3'}S(O)₂R_{3"}, -S(O)₂NR_{3'}R_{3"}, -NR_{3'}C(O)NR_{3"}R_{3‴}, -SR_{3'} , -S(O)R_{3'}, S(O)₂R_{3'}, -C(O)OR_{3'},-C(O)NR₃,R_{3"}, -OCH₂CH₂OH, -NR₃,S(O)₂NR_{3"}R_{3‴}, -C(CH₃)₂OR_{3'};
**R_{3'}**, **R_{3"}**, and **R_{3‴}** are independently selected from a hydrogen atom or branched or unbranched C₁₋₆ alkyl radical;
**n** is 0, 1, 2 or 3;
**m** is 0, 1, 2 or 3;
**q** is 0, 1, 2 or 3;
**r** is 0, 1, 2 or 3;
**s** is 0, 1, 2 or 3;
**i** is 0, 1, 2 or 3;
**j** is 0, 1, 2 or 3;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt, co-crystal or prodrug thereof, or a corresponding solvate thereof.

2. A compound according to claim 1, wherein **Rₐ** and **Rₐ** are a hydrogen atom.

3. A compound according to any one of claims 1 to 2 wherein **R₁** is a hydrogen atom; or -O**R₁ₐ**;

4. A compound according to claim 3 where **R₁ₐ** is branched or unbranched C₁₋₆ alkyl radical, preferably methyl.

5. A compound according to any one of claims 1 to 4 wherein **R₂** is a hydrogen atom; a branched or unbranched C₁₋₆ alkyl radical, preferably methyl or ethyl; a -(CH₂)ₙ-OR₂ₐ; a -C(O)R_{2b}; or an optionally substituted -(CH₂)_{q}-C₃₋₉ heterocycloalkyl.

6. A compound according to claim 5 wherein **R₂ₐ** is a hydrogen atom; a branched or unbranched C₁₋₆ alkyl radical optionally, preferably an ethyl unsubstituted or substituted with -OH.

7. A compound according to claim 5 where **R_{2b}** is a C₃₋₉ heterocycloalkyl radical, preferably a tetrahydrofuranyl.

8. A compound according to claim 5, where **R₂** represents one of the following moieties optionally substituted:

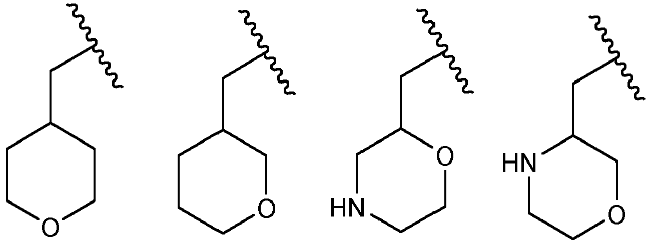

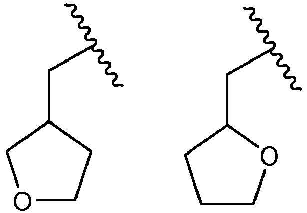

9. A compound according to any one of claims 1 to 8 wherein **R₃** represents one of the following moieties:

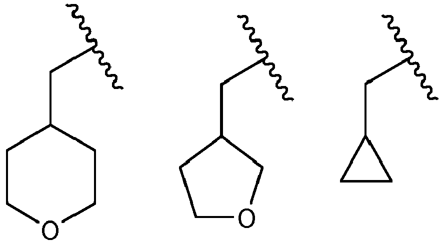

10. A compound according to claim 1 selected from:
**[1]** 2-(5-((3-(Cyclopropylmethyl)-2,4,5-trioxoimidazolidin-1-yl)methyl)-1,2,4-oxadiazol-3-yl)-N-(2-hydroxyethyl)-N-(2-methoxyphenyl)acetamide;
**[2]** 2-(5-((3-(Cyclopropylmethyl)-2,4,5-trioxoimidazolidin-1-yl)methyl)-1,2,4-oxadiazol-3-yl)-N-(3-methoxypyridin-4-yl)acetamide;
**[3]** 2-(5-((3-(Cyclopropylmethyl)-2,4,5-trioxoimidazolidin-1-yl)methyl)-1,2,4-oxadiazol-3-yl)-N-(pyridin-2-yl)acetamide;
**[4]** 2-(5-((3-(cyclopropylmethyl)-2,4,5-trioxoimidazolidin-1-yl)methyl)-1,2,4-oxadiazol-3-yl)-N-(3-hydroxypropyl)-N-(2-methoxyphenyl)acetamide;
**[5]** 2-(5-((3-(cyclopropylmethyl)-2,4,5-trioxoimidazolidin-1-yl)methyl)-1,2,4-oxadiazol-3-yl)-N-(2-(2-hydroxyethoxy)ethyl)-N-(2-methoxyphenyl)acetamide;
**[6]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-4-yl)methyl)acetamide;
**[7]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-3-yl)methyl)acetamide;
**[8]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide;
**[9]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-3-yl)methyl)acetamide;
**[10]** 2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydro-2H-pyran-2-yl)methyl)acetamide;
**[11]** N-(2-methoxyphenyl)-2-(5-((2,4,5-trioxo-3-((tetrahydro-2H-pyran-4-yl)methyl)imidazolidin-1-yl)methyl)-1,2,4-oxadiazol-3-yl)acetamide;
**[12]** N-(2-hydroxyethyl)-N-(2-methoxyphenyl)-2-(5-((2,4,5-trioxo-3-((tetrahydro-2H-pyran-4-yl)methyl)imidazolidin-1-yl)methyl)-1,2,4-oxadiazol-3-yl)acetamide;
**[13]** N-(2-methoxyphenyl)-2-(5-((2,4,5-trioxo-3-((tetrahydrofuran-3-yl)methyl)imidazolidin-1-yl)methyl)-1,2,4-oxadiazol-3-yl)acetamide;
**[14]** N-(2-hydroxyethyl)-N-(2-methoxyphenyl)-2-(5-((2,4,5-trioxo-3-((tetrahydrofuran-3-yl)methyl)imidazolidin-1-yl)methyl)-1,2,4-oxadiazol-3-yl)acetamide;
**[15]** (R)-2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide;
**[16]** (S)-2-(5-((3-(cyclopropylmethyl)-2,4,5-trioxoimidazolidin-1-yl)methyl)-1,2,4-oxadiazol-3-yl)-N-(2-methoxyphenyl)-N-((tetrahydrofuran-2-yl)methyl)acetamide;
**[17]** 2-(5-((3-(cyclopropylmethyl)-2,4,5-trioxoimidazolidin-1-yl)methyl)-1,2,4-oxadiazol-3-yl)-N-((5,5-dimethyltetrahydrofuran-2-yl)methyl)-N-(2-methoxyphenyl)acetamide;
**[18]** 2-(5-((3-(cyclopropylmethyl)-2,4,5-trioxoimidazolidin-1-yl)methyl)-1,2,4-oxadiazol-3-yl)-N-(2-methoxyphenyl)-N-(((2S,5S)-5-methyltetrahydrofuran-2-yl)methyl)acetamide;
**[19]** N-(2-(5-((3-(cyclopropylmethyl)-2,4,5-trioxoimidazolidin-1-yl)methyl)-1,2,4-oxadiazol-3-yl)acetyl)-N-(2-methoxyphenyl)tetrahydrofuran-2-carboxamide;
**[20]** 2-(5-((3-(cyclopropylmethyl)-2,4,5-trioxoimidazolidin-1-yl)methyl)-1,2,4-oxadiazol-3-yl)-N-(2-methoxyphenyl)-N-((5-oxotetrahydrofuran-2-yl)methyl)acetamide;
**[21]** 2-(5-((3-(cyclopropylmethyl)-2,4,5-trioxoimidazolidin-1-yl)methyl)-1,2,4-oxadiazol-3-yl)-N-((4-hydroxytetrahydrofuran-2-yl)methyl)-N-(2-methoxyphenyl)acetamide;
**[22]** 2-(5-((3-(cyclopropylmethyl)-2,4,5-trioxoimidazolidin-1-yl)methyl)-1,2,4-oxadiazol-3-yl)-N-(2-methoxyphenyl)-N-(morpholin-2-ylmethyl)acetamide;
**[23]** 2-(5-((3-(cyclopropylmethyl)-2,4,5-trioxoimidazolidin-1-yl)methyl)-1,2,4-oxadiazol-3-yl)-N-(2-methoxyphenyl)-N-(morpholin-3-ylmethyl)acetamide;
**[24]** 2-(5-((3-(Cyclopropylmethyl)-2,4,5-trioxoimidazolidin-1-yl)methyl)-1,2,4-oxadiazol-3-yl)-*N*-ethyl-*N*-(2-methoxyphenyl)acetamide;
**[25]** 2-(5-((3-(Cyclopropylmethyl)-2,4,5-trioxoimidazolidin-1-yl)methyl)-1,2,4-oxadiazol-3-yl)-*N*-phenylacetamide;
**[26]** 2-(5-((3-(Cyclopropylmethyl)-2,4,5-trioxoimidazolidin-1-yl)methyl)-1,2,4-oxadiazol-3-yl)-*N*-(2-methoxyphenyl)-*N*-methylacetamide; or
**[27]** 2-(5-((3-(Cyclopropylmethyl)-2,4,5-trioxoimidazolidin-1-yl)methyl)-1,2,4-oxadiazol-3-yl)-*N*-(2-methoxyphenyl)acetamide;
or a pharmaceutical acceptable salt, stereoisomer, co-crystal, prodrug or solvate thereof.

11. Process for the preparation of a compound of general formula **(I)**: 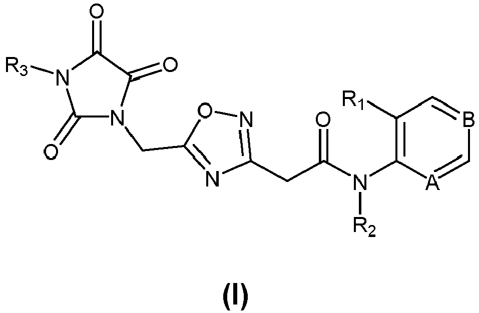
which comprises reacting a compound of formula (IX): 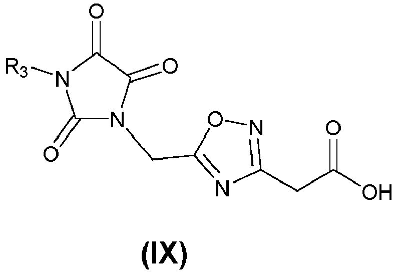
with a compound of formula (X): 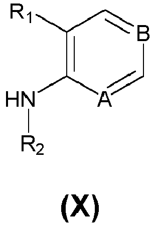
wherein **A, B, R₁, R₂ and R₃** are as defined in any of claims 1 to 9.

12. Process for the preparation of a compound of general formula **(I)**: 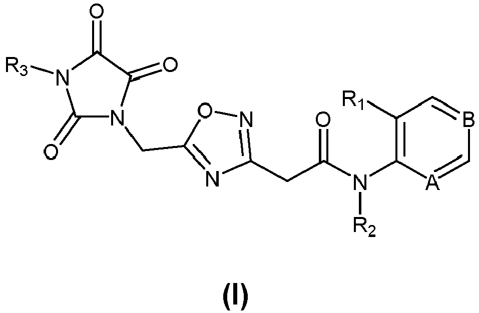
comprising the reaction between a compound of general formula (XIX): 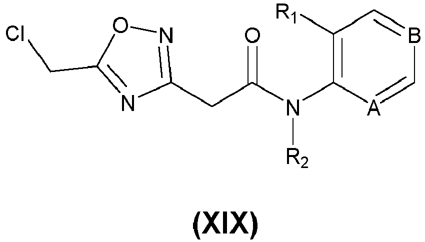
with a compound of formula (XIX): 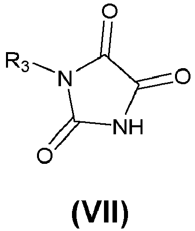
wherein **A, B, R₁, R₂** and **R₃** are as defined in any of claims 1 to 9.

13. A compound according to any one of claims 1 to 10 for use as a medicament.

14. A compound according to any one of claims 1 to 10, for use as antibiotic.

15. A compound according to claim 14 for use as antibiotic for the in the treatment and or prophylaxis of *Staphylococcus sp.* infections.

16. A compound for use according to claim 13 wherein the infection is an infection caused by *Staphylococcus aureus.*

17. A pharmaceutical composition comprising a compound according to any one of claims 1 to 10 or a pharmaceutically acceptable salt, isomer, co-crystal, prodrug or solvate thereof, and at least a pharmaceutically acceptable carrier, additive, adjuvant or vehicle.
